(19) Europäisches Patentamt
European Patent Office
Office européen des brevets

(11) EP 0 507 870 B1

(12) **EUROPEAN PATENT SPECIFICATION**

(45) Date of publication and mention of the grant of the patent: **10.04.1996 Bulletin 1996/15**

(21) Application number: **91902885.2**

(22) Date of filing: **12.12.1990**

(51) Int. Cl.$^6$: **A61K 38/42**, A61K 35/14, C07K 1/00, C07K 14/00, C07K 17/00

(86) International application number: **PCT/US90/07442**

(87) International publication number: **WO 91/09615 (11.07.1991 Gazette 1991/15)**

(54) **POLYHEMOGLOBIN STABILIZED BY PURINE DERIVATIVES AND GLUTATHIONE**

POLYHÄMOGLOBIN DURCH PURINDERIVATE UND GLUTATHION STABILISIERT

POLYHEMOGLOBINE STABILISEE A L'AIDE DE DERIVES DE PURINE ET DE GLUTATHION

(84) Designated Contracting States:
**AT BE CH DE DK ES FR GB GR IT LI LU NL SE**

(30) Priority: **29.12.1989 US 459071**

(43) Date of publication of application: **14.10.1992 Bulletin 1992/42**

(73) Proprietor: **TEXAS TECH UNIVERSITY HEALTH SCIENCES CENTRE**
**Lubbock, TX 79409 (US)**

(72) Inventors:
- **FEOLA, Mario**
  **Lubbock, TX 79416 (US)**
- **SIMONI, Jan, S.**
  **Lubbock, TX 79415 (US)**
- **CANIZARO, Peter, C.**
  **Lubbock, TX 79416 (US)**

(74) Representative: **Horner, Martin Grenville et al**
**Cruikshank & Fairweather**
**19 Royal Exchange Square**
**Glasgow G1 3AE Scotland (GB)**

(56) References cited:

| | |
|---|---|
| **US-A- 4 061 736** | **US-A- 4 401 652** |
| **US-A- 4 473 496** | **US-A- 4 831 012** |
| **US-A- 4 857 636** | **US-A- 4 876 241** |

- **Analytical Biochemistry, Volume 106, No. 1, issued 15 July 1980, REED et al., "High-Performance Liquid Chromatography Analysis of Nanomole Levels of Glutathione, Glutathione Disulfide, and Related Thiols and Disulfides", pages 55-62, see entire document.**
- **Analytical Biochemistry, Volume 157, No. 1, issued 15 August 1986, DELOACH et al., "A Continuous-Flow High-Yield Process for Preparation of Lipid-Free Hemoglobin", pages 191-198, see the Abstract and the Materials and Methods.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 507 870 B1

Printed by Rank Xerox (UK) Business Services
2.10.4/3.4

## Description

This invention relates to blood substitutes and methods for their preparation. More particularly, it relates to novel hemoglobin composition which is effective in sustaining life after severe hemorrhage in animals of various species, potentially in humans, and which is generally free of toxicity.

Blood absolves many factions, all vital. However, severe hemorrhage endangers life for two main reasons: (1) the drop in circulating blood volume reduces tissue perfusion (ischemia); and (2) the reduction in oxygen transport impairs tissue oxygenation (hypoxia). The circulatory system reacts to these changes by vasoconstriction, which further aggravates ischemia and hypoxia. Ultimately, alterations of cell metabolism and function develop, which lead to shock and death.

In this context, a "blood substitute" is not a preparation that can replace blood in all of its functions, but an emergency resuscitative fluid that is capable of (a) restoring blood volume, (b) transporting oxygen, and (c) reducing vasoconstriction. Obviously, this fluid must be free of toxic side-effects, as well as of agents of disease such as bacteria and viruses.

For over 50 years, efforts directed to the development of a blood substitute have focused on hemoglobin (Hb), because this is the only substance capable of picking up enough oxygen from atmospheric air to serve as a physiological oxygen carrier. In addition, hemoglobin exerts the same colloid-osmotic pressure as serum albumin and can, therefore, serve as a plasma volume expander. However, these efforts have not been successful due to a number of problems that have been slow to be recognized and difficult to resolve. These problems are: (1) toxicity due to the contamination of hemoglobin with (a) environmental bacterial endotoxins, (b) stromal phospholipids and (c) non-heme proteins and peptides; (2) high oxygen affinity of hemoglobin in solution which interferes with oxygen release to the tissues; (3) instability of the Hb molecule with tendency to extravasation and rapid renal excretion; (4) tendency of hemoglobin to undergo autoxidation with generation of met-Hb (non functional Hb) and toxic oxygen free-radicals; and (5) ability of Hb as a blood byproduct to transmit blood-related diseases, such as hepatitis and AIDS.

Historically, first to be recognized was the problem of toxicity, i.e., an ability on the part of Hb solutions to activate the intravascular coagulation of blood and cause damage to the kidney. Rabiner in the 1960's popularized the notion that such toxicity was due to the stroma of red blood cells (fragments of red cell membranes) rather than to Hb. He emphasized the need of a "stroma-free hemoglobin." However, this term has, over the years, belied the fact that a hemoglobin truly free of all stromal elements has not been produced. The toxic factors of the red cell membrane have been identified as the aminophospholipids phosphatidylethanolamine (PE) and - serene (PS), which normally reside on its cytoplasmic side (M. Feola et al., "Toxic factors in the red blood cell membrane," The Journal of Trauma, Volume 29: pages 1065-1075, 1989). It has been found that these compounds have a peculiar affinity for Hb (they are more difficult to remove from the Hb solution than other stromal components), and when Hb contaminated with PE and PS is infused into experimental animals (rabbits and monkeys) in significant volumes (at least 1/3 of the animal's calculated blood volume), it causes a "systemic inflammatory reaction" characterized by activation of intravascular coagulation and complement, activation of leukocytes and platelets, and development of ischemic-inflammatory lesions in the vital organs (M. Feola et al., "Toxicity of polymerized hemoglobin solutions," Surgery, Gynecology and Obstetrics, Volume 166: pages 211-222, 1988; M. Feola et al., "Complement activation and the toxicity of stroma-free hemoglobin solutions in primates," Circulatory Shock, Volume 25: pages 275-290, 1988).

A problem that only recently has been recognized is the easy contamination of Hb solutions with environmental bacterial endotoxins. Until development of the limulus amoebocyte lysate test, the U.S. pharmacopoeia relied on the rabbit pyrogenicity test as the assay for the detection of endotoxins. The above referenced articles have also reported that hemoglobin contaminated with endotoxins at concentrations well below the pyrogenicity level causes the same kind of toxicity as hemoglobin contaminated with aminophospholipids (the toxic component of endotoxin is in fact a lipid, "lipid A"). Bacterial endotoxins can be removed from biological solutions by use of affinity chromatography columns, such as Detoxi-Gel columns (Pierce Chemical Co.). However, these columns cannot remove all endotoxin if the starting material contains more than 2 endotoxin units per milliliter (as determined by use of the "quantitative chromogenic limulus test" (QCL-1000, Whittaker M.D., Bioproducts, according to which 1 EU = 0.1 nanograms of bacterial lipopolysaccharide).

Hemoglobin must be purified from non-heme proteins and peptides. While no toxicity has been related to the presence of any particular protein, purification is mandated by the necessity of reducing the immunogenicity of Hb solutions. Also, it has been hypothesized that a peptide is responsible for the vasoconstrictor effect of Hb solutions found in isolated organs (heart and kidney) and isolated arteries. A variety of methods for such purification are known to the art. These include: (1) centrifugation and filtration (Doczi, U.S. Patent No. 3,991,181); (2) toluene extraction (Bonsen et al., U.S. Patents No. 4,001,200 and No. 4,001,401; (3) ultrafiltration (Kothe et al., U.S. Patent No. 4,526,715); (4) ultrafiltration plus acid precipitation (Bonhard et al., U.S. Patents No. 4,136,093 and No. 4,336,248); (5) ion-exchange chromatography (Meiller, U.S. Patent No. 4,100,149); (6) zinc precipitation (Tye, U.S. Patents No. 4,473,494 and No. 4,529,719); and (7) crystallization (DeVenuto et al., Journal of Laboratory and Clinical Medicine, Volume 89: pages 509-514, 1977). However, methods 1-4 have intrinsic limitations as to the capability of completely separating Hb from other proteins, while methods 5-7 do not lend themselves to large-scale purification.

A problem recognized in the 1970's is the high oxygen affinity of Hb in solution. This is the property that regulates the ability of hemoglobin to pick up oxygen from air in the lungs and release it to the tissues. An expression of this property is the $P_{50}$ value (the partial tension of oxygen at which Hb is 50% saturated). The lower the $P_{50}$, the greater the ability of hemoglobin to bind oxygen, which translates into a reduced ability to unload it to the tissues. The $P_{50}$ of human blood is ~28 mm Hg, while the $P_{50}$ of human Hb in solution is ~13 mm Hg. The difference is due to the fact that within the red blood cell Hb reacts with 2,3-diphosphoglycerate (2,3-DPG), which reduces the affinity of Hb for oxygen. Outside the red blood cell, that interaction is lost. The consequence is that Hb binds $0_2$ so tightly that it ceases to function as an $0_2$ carrier. To resolve this problem, Benesch et al., developed a covalent reaction of Hb with pyridoxal-5'-phosphate, a "2,3-DPG analogue." It was at first hoped that such reaction would both reduce oxygen affinity and stabilize the Hb molecule in tetrameric form. However, this failed to materialize. Bovine hemoglobin in solution, however, has the same $P_{50}$ value as human blood, and its oxygen affinity is regulated by chlorides rather than by 2,3-DPG (M. Feola at al., "Development of a bovine stroma-free hemoglobin solution as a blood substitute," Surgery, Gynecology and Obstetrics, Volume 157: pages 399-408, 1983). Considering this favorable property, plus the large-scale availability of bovine RBCs and the low antigenicity of pure hemoglobin among mammals, there are advantages to the use of bovine hemoglobin as the basis for a blood substitute.

Another problem recognized in the 1970's is the rapid extravasation of hemoglobin with short intravascular persistence. This is generally attributed to a tendency of Hb tetramers, $\alpha_2\beta_2$, to dissociate to dimers, $2\alpha\beta$, which pass with greater ease through the blood capillaries. It now appears that the surface electric charge of the protein also plays an important role, with electronegativity and low isoelectric point favoring intravascular persistence. Hemoglobin extravasation has several undesirable effects: (1) the plasma volume-expanding effect is of short duration; (2) the passage of Hb through the renal glomeruli generates an osmotic diuretic effect which reduces, rather than sustains, plasma volume; (3) the reabsorption of Hb in the renal tubules causes injury to the tubular cells; and (4) the passage of Hb into the interstitial fluids causes edema and cell injury. The prior art has focused exclusively on the prevention of Hb dimerization. For this purpose, three types of Hb modification have been developed: (a) intermolecular crosslinking (polymerization); (b) conjugation of Hb with other molecules; and (c) intramolecular crosslinking of $\alpha$ or $\beta$ chains. The most widely used method is the intermolecular cross-linking of Hb with glutaraldehyde (Bonsen et al., U.S. Patents No. 4,001,200, No. 4,001,401 and No. 4,053,590; Morris et al., U.S. Patent No. 4,061,736; Bonhard et al., U.S. Patent No. 4,136,093). However, there are several problems with this method: (1) glutaraldehyde is intrinsically toxic, and the potential toxicity of its metabolic byproducts is unknown; (2) the compound is very reactive and tends to form multiple bridges with various sites ($\alpha$- and $\varepsilon$-amino groups and sulfhydryl groups) of the Hb molecule, leading to the formation of unpredictable numbers of molecular species; (3) the polymerization process is difficult to control and appears to continue during storage at 4°C, leading to the formation of progressively larger polymers of increased viscosity and oxygen affinity; (4) the non-specific nature of the crosslinking may still allow the presence of Hb dimers in the solution. As an alternative, Hb has been coupled with large-size molecules, such as dextran and hydroxyethyl starch (U.S. Patent No. 4,064,118), polyethylene or polypropylene glycols (U.S. Patent No. 4,412,989), inulin (U.S. Patent No. 4,377,512), and poly-alkylene oxide (U.S. Patent No. 4,670,417). However, these conjugated hemoglobins have increased oxygen affinity and tend to acquire unfavorable properties peculiar to the coupling substances. Intramolecular crosslinking has been achieved by use of "diaspirin" esters (Type, U.S. Patent No. 4,529,719; Walder, U.S. Patent No. 4,598,004) and "periodate-oxidized adenosine triphosphate" (o-ATP) (F. J. Scannon, "Molecular modification of hemoglobin," Critical Care Medicine, Volume 10: pages 261-265, 1982; A.G. Greenburg and P.W. Maffuid, "Modification of hemoglobin - Ring opened diols," Advances in Blood Substitute Research, Alan R. Liss, New York, 1983: pages 9-17). However, the diaspirin-hemoglobins still have short intravascular persistence (half-life of 3-4 hours), while the ATP-hemoglobins have been found unsatisfactory due to high levels of met-Hb and high oxygen affinity combined with short half-life.

Significant progress has been reported by investigators who have reacted human Hb with pyridoxal-5'-phosphate and glutaraldehyde, to yield polymerized pyridoxalated Hb ("poly-PLP-hemoglobin"), i.e., a hemoglobin allegedly with both low oxygen affinity and prolonged intravascular persistence (G.S. Moss et al., "Hemoglobin solution - From tetramer to polymer," Biomaterials, Artificial Cells and Artificial Organs, Volume 16(1-3): pages 57-69, 1988; F. DeVenuto and A. Zegna, "Preparation and evaluation of pyridoxalated-polymerized human hemoglobin," Journal of Surgical Research, Volume 34: pages 205-212, 1983). However, it has been found that pyridoxalation interferes with polymerization. Thus, it happens that pyridoxalated Hb with reduced $0_2$ affinity is rapidly excreted via the kidney, while polymerized Hb with prolonged half-life has high oxygen affinity. In conclusion, th eproblem of Hb stabilization without compromise of oxygen transport function has not thus far been resolved.

Over the past few years, questions have been raised concerning an intrinsic toxicity of hemoglobin. Experimental observations have been reported of a vasoconstrictor effect. It has also been speculated that, since Hb tends to autoxidize to met-Hb (i.e., the oxidation of the heme iron from the ferrous +2 to the ferric +3 state, a process that generates toxic oxygen free-radicals), it might act as a pro-oxidant when infused into the circulation. This effect would produce lipoperoxidation of cell membranes and cause injury to cell structures. both these effects, vasoconstriction and generation of oxygen free-radicals, would aggravate, rather than alleviate, the ischemic-hypoxic injuries caused by hemorrhage. The results of the experimental studies have been reported which show that vasoconstriction and generation of radicals can

both be controlled by three steps: (1) complete purification of Hb; (2) preparation and stabilization of the Hb molecule with low levels of met-Hb formation; and (3) addition of oxygen radical-scavengers (M. Feola et al., "Biocompatibility of hemoglobin solutions. I. Reactions of vascular endothelial cells to pure and impure hemoglobins," Artificial Organs, Volume 13(3): pages 209-215, 1989).

Finally, Hb solutions carry the risk of blood product-transmissible diseases. While bacteria and parasites can be easily removed by filtration or ultrafiltration, viruses represent a more serious problem. Two methods of virus inactivation are known to the art. One is a physical method, which consists of pasteurization of hemoglobin in deoxy- form at 60°C and pH 7.5 for 10 hours. This method has been found to inactivate model viruses (sindbis, polio, pseudorabies) as well as the human immunodeficiency virus (HIV) (T.N. Estep et al., "Virus inactivation in hemoglobin solutions by heat," Biomaterials, Artificial Cells and Artificial Organs, Volume 16(1-3): pages 129-134, 1988). The other is a chemical method that consists of treatment with chloroform (S.M. Feinston et al., "Inactivation of hepatitis B virus and non-A non-B hepatitis by chloroform," Infection and Immunity, Volume 41: pages 816-821, 1983). However, both methods produce significant denaturation of hemoglobin, unless special measures are taken.

The present invention is a composition for use as a blood substitute comprising a hemoglobin reacted with O-ATP and o-adenosine to form a crosslinked product, and reacted with a quantity of reduced glutathione, the product being dissolved in a saline solution. In one embodiment the composition comprises mammalian (preferably bovine) hemoglobin (Hb), which is: (a) totally and completely purified; (b) crosslinked intramolecularly with periodate-oxidized ATP (o-ATP) and intramolecularly with periodate-oxidized adenosine (o-adenosine); (c) reacted with reduced glutathione (GSH); and (d) dissolved into an electrolyte-balanced saline solution enriched with magnesium chloride ($MgC1_2$) and mannitol. Since o-ATP and o-adenosine are two purine (P) derivatives, the product is denoted herein as Hb-PP-GSH.

Preferably the hemoglobin preparation combines the favorable properties of its constituents: (1) it is an effective oxygen-carrier, in that bovine Hb has a naturally low oxygen affinity ($P_{50}$ value of 28 mm Hg) that is not affected by the various chemical reactions; (2) it is an effective plasma volume expander by virtue of the fact that hemoglobin crosslinked both intra- and inter- molecularly has prolonged intravascular persistence (half-life of 24 hours); (3) it has vasodilating properties due to the fact that both purines relax norepinephrine-induced vasoconstriction; and (4) it does not exert a pro-oxidant effect, due to the presence of reduced glutathione and mannitol.

The favorable properties of bovine hemoglobin have been demonstrated (M. Feola et al., "Development of a bovine stroma-free hemoglobin solution as a blood substitute," Surgery, Gynecology and Obstetrics, Volume 157: pages 399-408, 1983). Aside from its large-scale availability and the avoidance of transmissible diseases peculiar to human blood (AIDS in particular), bovine Hb has a $P_{50}$ value more than double that of human Hb (28 versus 13 mm Hg) and does not need 2,3-DPG modulation. In accordance with a preferred feature of the present invention, the oxygen affinity of bovine Hb can be further lowered by increased concentrations of chloride ions. With regard to potential immunological problems, the feasibility of Hb tranfusions across different mammalian species has been well-demonstrated. In fact, pure bovine hemoglobin has been administered repreatedly (up to 6 times) to rabbits and monkeys in volumes corresponding to 1/3-1/2 of calculated blood volumes without clinical evidence of reaction and without formation of antibodies detectable by Ouchterlony's test (M. Feola et al., "Immunologic biocompatibility of hemoglobin solutions," Trasfusione de sangue (Italian), Volume 33: pages 121-128, 1988).

The present invention also provides a method for extracting purified hemoglobin from whole blood, comprising the steps of:

centrifugation of the whole blood to remove platelets and plasma and suspending the resulting leukocyte-erythrocyte mixture in solution;

cooling the leukocyte-erythrocyte mixture to aggregate the leukocytes;

removing the leukocyte aggregate by filtration;

extracting hemoglobin from the erythrocytes by dialyzing against a hypotonic solution, and subjecting the erythrocytes to ultrafiltration under increased hydrostatic pressure;

converting the hemoglobin to carboxy form by saturating the hemoglobin with carbon monodoxide;

pasteurizing the hemoglobin solution to denature and precipitate non-heme proteins;

centrifuging the hemoglobin solution mixed with chloroform to remove phospholipids and precipitated non-heme proteins;

removing any remaining chloroform from the supernatent hemoglobin solution by flusing with carbon monoxide gas; and

removing endotoxins from the hemoglobin solution by passing the solution through an affinity chromatography column.

In order to produce a Hb solution free of bacterial endotoxins, the strategy preferably used in the present method of preparation is that of preventing, rather than correcting contamination. Given the affinity of endotoxin for hemoglobin, once significant contamination has occurred, purification is extremely difficult, if not impossible, to attain. Prevention requires that: (1) the starting material be minimally contaminated; (2) the preparative steps be carried out in closed system fashion; (3) all surfaces coming in contact with Hb be sterile and pyrogen-free; (4) all chemicals be pure; (5) all solutions be sterile and pyrogen-free; and (6) quality control be instituted at every step. The most sensitive method for

the detection endotoxin is the "quantitative chromogenic limulus test" (QCL-1000, Whittaker Bioproducts). If the starting material, or hemoglobin at any preparative step is found to contain more than 2 EU/ml, it is discarded. By maintaining a low level of contamination throughout the process, complete purification can be achieved by final passage of the solution through an affinity chromatography column, such as the Detoxi-Gel (Trade mark) (Pierce Chemical Company).

The same principle of avoidance of gross contamination coupled with final purification is applied to the removal of stromal phospholipids (aminophospholipids in particular). To obviate stromal contamination, the present method adapts a method of red blood cell dialysis and ultrafiltration originally presented by J.R. DeLoach et al., in Analytical Biochemistry, Volume 157: pages 191-198, 1986. According to a preferred embodiment, the RBCs (erythrocytes) are first dialyzed against a hypotonic phosphate solution until the suspension reaches an osmolarity of 160 mOsm/ℓ. At this point, the RBCs assume a spherical shape and the pores of the cell membrane are stretched. The cells are then subjected to ultrafiltration through 0.1 µm pore Amicon (Trademark) filters under 7.031 x $10^3$ kg/$m^2$ (10 psi) column pressure. Thus, hemoglobin is "squeezed out" of the cells without disruption of the cell membranes. In accordance with the present invention, a single closed-system process is used for both RBC dialysis and ultrafiltration, which is sterile, pyrogen-free and disposable. In additional, the dialysis fluid consists of sterile, pyrogen-free deionized water adjusted to a pH of 8.2 with Tham (Trademark) solution, instead of a phosphate solution, which reduces hemoglobin oxidation. The result of this process is a Hb solution that contains approximately 3-5 mg/dl of phospholipids (measured by the "phospholipid test set," Boehringer-Mannheim Diagnostics, Indianapolis, Indiana), with only traces of the aminophospholipds PE and PS (as determined by thin-layer chromatography). These residual phospholipids are removed by chloroform extraction. Because of the low level of phospholipids present, this step can be carried out by use of low concentrations of chloroform for short-time centrifugations. Thus, the denaturation of hemoglobin is prevented.

The same principle applies to a further embodiment, namely the purification of hemoglobin from non-heme proteins and peptides. In this embodiment, the first step is represented by the removal of all plasma proteins during the "purification" of red blood cells. Second, the method of Hb-extraction from RBCs without large-scale disruption of red cell membranes prevents contamination with stromal proteins. Finally, purification is achieved by "selective thermal precipitation" (see: P.A. Belter, E.L. Cussler, W.S. Hu, editors, Bioseparations, John Wiley & Sons, New York, 1988: pages 227-229). The scientific basis of this method rests on the fact that the denaturation (and precipitation) of proteins by temperature follows first-order chemical kinetics with an Arrhenius temperature dependence:

$$\frac{d|P|}{dt} = -\kappa|P|$$

where P is the dissolved protein concentration. The rate constant $\kappa$ is given by:

$$K=\kappa_0 e^{E/RT}$$

where $\kappa_0$ is a characteristic constant and is the "activation energy of denaturation" and T is temperature. The energy of denaturation varies from one protein to another. Because E appears exponentially in the equation, it has a large effect when temperature is even slightly changed. This energy is also affected by changes in pH. We have also found that hemoglobin saturated with carbon monoxide (HbC0) is resistant to temperature-induced precipitation at pH 7.6-7.8. We have found that pasteurization at 60°C for 9 hours, followed by pasteurization at 70°C for 1 hour, at pH 7.6-7.8, of a solution of hemoglobin in carboxy form at the concentration of 10 g/dl, precipitates all non-heme proteins with little denaturation of hemoglobin. The absence of non-hemoglobin proteins in the solution as prepared by the present method has been verified by use of isoelectric focusing (IEF) and by size-exclusion and anionic-exchange high pressure liquid chromatography (HPLC). The non-denaturation of recovered hemoglobin is demonstrated by the absence of "smudging" of focused bands on IEF and by the preservation of oxygen transport function (oxygen dissociation curves, $P_{50}$, Bohr effect).

Generally a byproduct of the purification process is the inactivation of viruses. In fact, chloroform extraction has been found to inactivate a number of lipid-enveloped viruses, such as hepatitis B, non-A non-B hepatitis, vaccinia and pox viruses, both in plasma and serum (Feinston et al., referenced above). Some viruses without lipids (Reoviruses) may also be partially inactivated with chloroform. On the other hand, it has been demonstrated that pasteurization at 60°C for 10 hours inactivates a number of non-lipid enveloped viruses, as well as the human immunodeficiency virus (HIV) (T.N. Estep et al., referenced above).

The present invention further provides a method for preparing a composition as decribed herein for use as a blood substitute, comprising the steps of:

converting a solution of purified hemoglobin to the carboxy form by saturating the hemoglobin with carbon monoxide;

dialyzing the resulting carboxy-hemoglobin solution against a normotonic solution to concentrate the hemoglobin to approximately 10 g/dl;

reacting the carboxy-hemoglobin with oxidized adenosine triphosphate (o-ATP) to effect predominantly intramolecular crosslinking among the hemoglobin molecules;

reacting the carboxy-hemoglobin with oxidized adenosine (o-adenosine) to effect predominantly intermolecular crosslinking between the hemoglobin molecules;

quenching the o-adenosine crosslinking reaction by adding reduced glutathione to the solution; and

converting the hemoglobin from the carboxy- to the oxy- form by flushing the solution with oxygen.

Because of the undesirable effects of polymerization with glutaraldehyde, the present method "stabilizes" the Hb molecule in tetrameric form by use of the dialdehyde derivative of adenosine 5'-triphosphate (o-ATP). The ATP molecule has three basic components: the purine base adenine, the sugar D-ribose and a triphosphate chain:

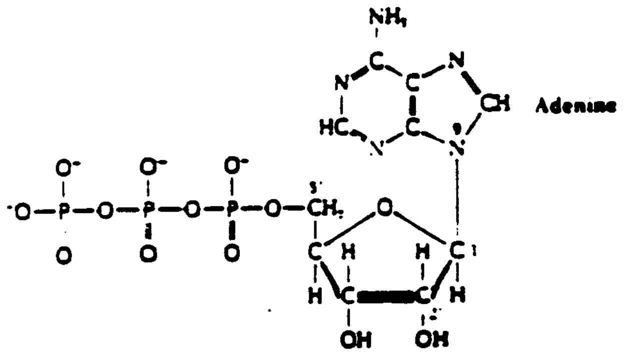

Adenosine triphosphate (ATP)

Oxidation of ATP with sodium periodate opens the ribose ring at the 2' ,3'-cis site and transforms the 2' ,3'-diol into the corresponding dialdehyde (P.N. Lowe et al., "Preparation and chemical propeties of periodate-oxidized adenosine triphosphate and some related compounds," Biochemical Society Transactions, Volume 7: pages 1131-1133, 1979):

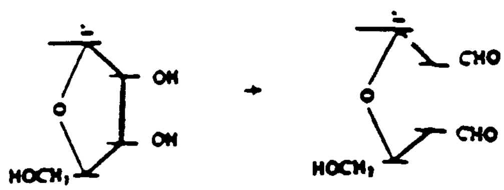

Each aldehyde group of the o-ATP molecule can react with the ε-amino group of lysine, to form a Schiff base adduct:

$$(HB)\text{-}NH_2 + OCH\text{-}ATP \rightarrow (HB)\text{-}N = CH\text{-}ATP$$

Since the 2,3-DPG pocket of hemoglobin (the region within the Hb molecule that binds 2,3-DPG) contains two lysines, it is possible to use o-ATP to crosslink these groups, thus stabilizing the molecule in tetrameric form. The presence of the triphosphate chain increases the specificity of this reaction, such specificity having been demonstrated for other polyphosphates such as pyridoxal-5'-phosphate. The advantage of ATP over other compounds is provided by the adenine moiety. In vivo, the hydrolysis of ATP to ADP, AMP and finally to adenosine has been found to produce beneficial pharmacologic effects, mainly vasodilation both in the systemic and pulmonary circulation. Additional beneficial effects have been demonstrated when ATP is given in combination with magnesium chloride ($MgCl_2$) in the setting of hemorrhagic shock. These include improvement of the microcirculation, improvement of cell membrane function and a "priming" effect on the restoration of intracellular adenine nucleotides (I.H. Chaudry and A.E. Baue, "Overview of Hemorrhagic shock," Pathophysiology of shock, anoxia and ischemia, R.A. Cowley and B.F. Trump, editors, Williams and Wilkins, Baltimore, Maryland, 1982: pages 203-219).

As noted above, previous attempts at crosslinking Hb with o-ATP have not been successful because the chemical reaction produces unacceptable levels of met-Hb (up to 30%), and Hb modified with o-ATP still has short intravascular persistence. In addition, ATP has an undesirable tendency to chelate divalent cations from the vascular system.

We have found that the oxidant effect of o-ATP is due to traces of iodate ($IO_4^-$ and $IO_3^-$) present in the compound. In fact, complete purification of o-ATP (see Example I) corrects that problem. We also found that met-Hb formation can be minimized by reacting o-ATP with carboxy-Hb (Hb saturated with carbon monoxide) rather than with deoxy-Hb, as has previously been done. We have discovered that the reaction of o-ATP with carboxy-Hb will take place if the pH of

the solution is reduced to about 7.20. With regard to the cation-chelating effect, we confirmed the report by Chaudry and Baue (see above) that preferably the addition of magnesium chloride ($MgC1_2$) in amounts equimolar with ATP eliminated that problem. There is left, however, the problem of short intravascular persistence. We found that Hb crosslinked intramolecularly, in tetrameric form, would still be filtered through the renal glomeruli and cause injury to the renal tubules. Therefore, it was necessary to crosslink hemglobin inter-, as well as intra-, molecularly if adequate intravascular retention times are to be attained.

The present invention utilizes a second purine derivative, the dialdehyde derivative of adenosine, or periodate-oxidized adenosine (o-adenosine), as a second crosslinking agent. Since the Hb molecule carries 44 lysine amino-groups on its surface, it is possible to use o-adenosine to bridge two or more of these groups and bind two or more Hb tetramers. The advantages of adenosine over other compounds are several. First, due to the presence of adenine, adenosine has a vasodilator effect similar to that of ATP (C. Su, "Extracellular functions of nucleotides in heart and blood vessels," Annual Review of Physiology, Volume 47: pages 665-676, 1985). In addition, adenosine inhibits platelet aggregation and improves glomerular filtration in the kidney, both effects being beneficial after hemorrhage and reperfusion (R. M. Berne: Regulatory Functions of Adenosine, Martin Nijhoff Publisher, Boston, Massachusetts, 1983). The reaction of hemoglobin with o-adenosine is unknown to the previous art. It is also important that the reaction be carried out with hemoglobin in the carboxy form in order to reduce met-Hb formation. Finally, the reaction proceeds very slowly at 4°C, so it can be stopped at any time after the formation of the desired molecular aggregate. This characteristic allows the preparation of Hb polymers of different molecular sizes in a planned, reproducible fashion (which cannot be done with the use of other crosslinking agents such as glutaraldehyde). The crosslinking of hemoglobin with o-adenosine is stopped by the addition of reduced glutathione (GSH), which, like lysine, carries an $\varepsilon$-amino group. By entering this reaction, GSH becomes part of the Hb composition.

The choice of GSH is based on the knowledge that this compound is abundant within the red blood cell, where its primary function is to work as an "oxidant trap" that protects hemoglobin from oxidant stress (A. Larsson: Functions of Glutathione: Biochemical, Physiological, Toxicological and Clinical Aspects, Raven Press, New York, 1983). We have verified that GSH protects hemoglobin in solution as well as within the erythrocytic environment. It has also been found that the crosslinking with o-adenosine followed by the reaction with GSH produces an increase of electronegative charges on the surface of the Hb molecule with reduction of Hb's isoelectric point from 6.8 to 6.1-6.2. This contributes to the stabilization of hemoglobin, in the sense that it prolongs intravascular persistence and prevents filtration through the kidney.

o-ATP and o-adenosine can be obtained from commercial sources (Sigma Chemical Co., St. Louis, Missouri) or can be prepared according to the methods described below as Example I and Example II. Reduced glutathione is obtained from a commercial source.

Following these reactions and the reconversion of carboxy-Hb to oxy-Hb, the new compound (Hb-PP-GSH) is dissolved into an electrolyte balanced saline solution preferably enriched with magnesium chloride ($MgC1_2$) and preferably also mannitol. $MgC1_2$ is added in an amount equimolar with ATP. This has several beneficial effects: (1) it controls the divalent cation-chelating effect of ATP; (2) it complements ATP in its beneficial effects on the microcirculation; and (3) it provides an excess of chloride ions which modulates downwards the affinity of Hb for oxygen (helps to maintain a high $P_{50}$ value). Mannitol is added in small amounts, based on the knowledge that it works as a scavenger of OH radicals (the most toxic oxygen-derived free-radicals), and perhaps of other radicals as well (B. A. Freeman and J.D. Crapo, "Free radicals and tissue injury," Laboratory Investigation, Volume 47: pages 412-426, 1982).

Thus, preferred embodiments of the present invention provide a chemical compound useful as a blood substitute, i.e., capable of (a) restoring and sustaining plasma volume, (b) supplying the vital organs with oxygen, and (c) relieving vasoconstriction after hemorrhage.

The present invention is able to provide such a blood substitute which is free of toxicity.

Other objects, features and advantages of the invention will become evident in light of the following detailed description of a preferred exemplary embodiment according to the present invention, which is described by way of example only.

Figure 1 shows a Spectrum Analysis of pure bovine hemoglobin obtained by HPLC with size-exclusion column. Chromatogram shows single peak located at 9.4 minutes, identifying hemoglobin in tetrameric form (64,000 daltons).

Figure 2 shows a Spectrum Analysis of pure bovine hemoglobin obtained by HPLC with DEAE column. Chromatogram shows several peaks located between 20 and 36 minutes, corresponding to different isoelectric points of various Hb components.

Figures 3A-B show the Spectrum Index of bovine hemoglobin before (3A) and after (3B) purification by pasteurization (HPLC with DEAE column, spectrum wave-length 230-599 nm). In Fig. 3A, non-Hb proteins are visible, located at retention times 17 and 51 minutes. In Fig. 3B, non-Hb proteins are no longer visible.

Figure 4 shows a Spectrum Analysis by HPLC size exclusion of bovine Hb crosslinked intramolecularly with o-ATP and intermolecularly with o-adenosine, and combined with reduced glutathione. Chromatogram shows a Hb molecular aggregate containing six peaks.

Figure 5 shows Spectrum Analysis by HPLC-DEAE column of bovine Hb modified as in Fig. 4. Chromatogram shows single peak at retention time 51 minutes. The isoelectric point of Hb has shifted (as compared to unmodified Hb) due to increase in surface electronegative charges.

Figure 6 shows examination by isoelectric focusing (IEF - Pharmacia).

Figure 7 shows the absorbance at 258 nm of successive fractions of the o-ATP and soldium periodate reaction mixture eluted from a Sephadex column with water; and

Figure 8 shows the absorbances at 258 and 232 nm of successive fractions of the o-adenosine and sodium periodate reaction mixture eluted from an anion exchange column with Eluent A.

## Example 1

The preferred process for preparing the complex product according to this invention comprises five steps: (A) purification of red blood cells; (B) extraction of hemoglobin; (C) purification of hemoglobin; (D) modification of hemoglobin (reaction with o-ATP, o-adenosine and glutathione); and (E) preparation of final product $(Hb\text{-}PP\text{-}GSH)_n$.

A. Purification of red blood cells (RBCs)

The preferred starting material is represented by bovine blood, for the reasons discussed above. However, the method of preparation can be used starting from other mammalian blood, including human. Bovine blood can be obtained from multiple healthy donors or from individual animals cleared for the slaughterhouse. In the first case, an adult cow is restrained, the neck is shaved and the skin prepared with antiseptic soap. Blood is drawn by puncture of the external jugular vein under aseptic conditions. Approximately 1,500 ml of blood can be obtained from one animal, collected into a 2-liter evacuated, sterile, pyrogen-free bottle, containing 200 ml of ACD anticoagulant (Virbac, Inc., Lenexa, Kansas). In the second case, after the animal is stunned prior to slaughtering, one side of the neck is quickly "prepared" and a trocar is inserted percutaneously into the carotid artery. Approximately 10 liters of blood can be removed from each adult cow. Blood from different animals is not mixed. The bottles are kept on ice in transit to the laboratory.

It is important, particularly when starting from bovine blood, that the RBCs (erythrocytes) be completely separated from white blood cells (leukocytes), platelets and plasma. This step reduces the load of non-heme proteins and other substances from which hemoglobin needs to be ultimately purified. Also, the removal of all leukocytes removes the viruses associated with these cells, lymphocytes in particular (cytomegalovirus, human immunodeficiency virus and others).

The RBCs are purified by a "spin-cool-filter" method. The "spin" step consists of multiple centrifugations carried out in closed-system fashion by use of a blood bank cell separator, such as the DIDECO system (Electromedics Inc., Englewood, Colorado), in the following manner:

1. Centrifugation at 1,100 rpm, at 15°C, for 20 minutes, to remove platelets and plasma;
2. Centrifugation at 4,500 rpm, at 15°C for 10 minutes for the more complete removal of plasma;
3. Washing (x 4) with isotonic saline solution (RBCs/saline 1:4) by centrifugation at 4,100 rpm, at 4°C, for 10 minutes.
4. Final washing with isotonic Tham (Trademark) solution, pH 8.1-8.2 (Tham USP, Abbott Laboratories, North Chicago, Illinois). This allows the suspension of washed RBCs into an electrolyte-free, high-pH solution, which protects the hemoglobin from oxidation.

For the "cool" part of the process, the RBCs are stored within "transfer packs" (sterile, pyrogen-free plastic containers made by Fenwal Laboratories, Deerfield, Illinois) at 4°C overnight, or for 18 hours. At low temperature, the white blood cells tend to aggregate into small clumps. For the "filter" step, the cells are passed through a 20 µm cellulose filter, such as the "high capacity transfusion filter" made by Fenwal, which removes the leukocyte aggregates.

To ascertain the absence of leukocytes and platelets, cell counts are carried out by use of a Coulter cell counter, and the absence of proteins in the suspension is verified by routine chemical methods. The presence of bacterial endotoxins is determined by the use of the "quantitative chromogenc limulus test" (QCL-1000, Whittaker Bioproducts, Walkersville, Maryland).

B. Extraction of hemoglobin.

The extraction of hemoglobin from RBCs is carried out in two steps. First, one liter of RBCs suspended into isotonic Tham (Trademark) solution, pH 8.1-8.2, at the concentration of 20% (hematocrit 0.20) is dialyzed against 10 liters of hypotonic (230 mOsm/ℓ) Tham (trademark) solution by means of an artificial kidney with 0.20 µm porosity, such as the "Krosflo II Filtration Module with 10 $Ft^2$ Surface Area" (Microgon Inc., Laguna Hills, California). The dialysis is carried out until the dialysate becomes reddish in color (hemoglobin tinged). At this point, the RBCs are swollen to a shperical shape, and the stretched cell membranes become permeable to Hb. As second step, a $7.031 \times 10^3$ $kg/m^2$ (10 psi)

pressure is applied to the artificial kidney, squeezing the Hb out of the cells without disruption of cell membranes. The membrane "ghosts" are discarded after one pass. As hemoglobin enters the hypotonic solution reservior, volume is maintained in the RBC reservoir by the addition of Tham (Trademark) solution, 230 mOsm/ℓ. The extracted hemoglobin is filtered through a 0.20 μm filter, such as the "Posidyne (Trademark) I.V. Filter" (PALL Biomedical Inc., Fajardo, Puerto Rico), to remove residual particulate debris or microbial contaminants, and stored in "transfer packs" at 4°C.

The result of this process is a Hb solution that contains 3-5 mg/dl of phospholipids (measured by use of the "phospholipid test set," Boehringer-Manmheim Diagnostics, Indianapolis, Indiana), with only traces of aminophospholipids PE and PS (determined by thin-layer chromatography).

C. Purification of hemoglobin.

This is carried out in four steps:

**1. Pasteurization of hemoglobin in carboxy form (HbCO).** This is carried out within a pre-sterilized, pyrogen-free biological reactor, such as the "Microlift-15 liter sterilizable-in-place bioreactor with NBS Model ML-4100 control system" (New Brunswick Scientific Co., Edison, New Jersey). This is a closed container with multiple entry sites for gases and liquids, ports for sampling, an agitator for stirring and temperature controls. The bioreactor is installed under an exhaust "fume hood." The hemoglobin is saturated with carbon monoxide (99.99% purity, Criogenic Rare Gas Co., Hanahan, South Carolina) by sequential flushing with sterilized gas at 760 mm Hg, 4°C, with slow agitation. Total saturation is verified by use of a cooximeter (Model 282, Instrumentation Laboratories, Lexington, Massachusetts). The process takes approximately 15 minutes. The solution is left under CO at 760 mm Hg. Pasteurization is then carried out by gradually raising the temperature within the bioreactor from 4 to 60°C and leaving it at that level for 9 hours, then raising it to 70°C for 1 hour. After these intervals, the temperature is gradually lowered back to 4°C.

**2. Centrifugation with chloroform.** For this step, the Hb solution is filtered through a 0.20 μm filter into 250-ml sterile, pyrogen-free centrifuge bottles sealed with appropriate caps ("polyallomer" bottles resistant to chloroform, proteins and alcohol obtained from Sorvall Division, Du Pont Co., Wilmington, Delaware).

A series of three centrifugations is carried out using a Sorvall centrifuge (Model -0TD75B with rotor TFA 20.250), in the following manner:

a. Centrifugation of hemoglobin mixed with chloroform in the ratio 15:1 (for each bottle: Hb, 232 ml; chloroform, 18 ml), at 760 x g, at 4°C, for 30 minutes. The supernatant is passed into a second series of bottles using sterile pyrogen-free tubing and a peristaltic pump, under laminar flow hood.

b. Centrifugation of Hb mixed with chloroform in the ration 16:1 at 1,600 x g, 4°C, for 15 minutes, and at 3,800 x g for 15 minutes. The supernatant is transferred into a third series of bottles.

c. Centrifugation without chloroform at 61,400 x g for 60 minutes.

After the third centrifugation, the Hb solution is transferred into 1000-ml sterile, pyrogen-free, evacuated bottles (Abbott Laboratories) with stirring bars, wherein remaining traces of chloroform are removed from the solution by flushing with sterilized CO gas, with slow stirring, at 4°C, for 2 hours.

The chloroform used for this step is HPLC grade with UV cutoff 244 nm (Fisher Schientific Co., Fair Lawn, New Jersey). The bottles are reusable following the treatment with (a) E-TOXA-Clean soap (Sigma Chemicals), (b) ethanol 190 proof, and (c) sterilization at 120°C for 80 minutes.

This series of centrifugations not only removes all phospholipids, but also separates the purified hemoglobin from the non-heme proteins that denatured and precipitated in the previous step of pasteurization.

**3. Filtration through endotoxin affinity-chromatography column.** The Hb solution is passed through an affinity chromatography column, such as the Detoxi-Gel (Trademark) column (Pierce Chemical Co., Rockford, Illinois), using inlet and outlet "transfer packs" and a peristaltic pump, thus creating a closed system. The procedure is carried out under a Class 100 laminar flow hood.

By this step, the concentration of endotoxin can be reduced from 2.0-2.5 EU/ml to < 0.10 EU/ml.

**4. Dialysis.** The Hb solution is dialyzed in a ratio 1:10 against sterile, pyrogen-free, deionized water, adjusted to a pH of 7.20 by the addition of Tham (Trademark) solution. The dialysis is carried out by use of an artificial kidney with 6,000-dalton porosity, such as the "90 SCE - Artificial Kidney" (C-DAK Co., Miami Lakes, Florida). This step: (a) eliminates small molecules, (b) concentrates hemoglobin to 10 g/dl, and (c) lowers the pH of the Hb solution from approximately 8.2 to approximately 7.2.

At this point in the process, "pure" hemoglobin has been produced, i.e., hemoglobin completely free of (a) bacterial endotoxins, (b) stromal lipids and phospholipids, and (c) non-heme proteins. Also, repeated filtrations through 0.20 μm filters at various points in the process are expected to have eliminated all microbial contaminants, while pasteurization and chloroform treatment are expected to have inactivated both non lipid- and lipid-enveloped viruses. Furthermore, the

use of hemoglobin in the carboxy- form allows its purification with a low degree of oxidation (1-2.5% met-hemoglobin formation).

D. Modification of hemoglobin.

The reaction of hemoglobin with o-ATP, o-adenosine and reduced glutathione is carried out within the biological reactor as follows. Hemoglobin in the carboxy-state, 10 g/dl in water adjusted with Tham (Trademark) to a pH of 7.20, is reintroduced into the bioreactor and kept at 4°C, with slow stirring, under one atmosphere of carbon monoxide.

o-ATP, prepared according to Example III and stored in powder form, is dissolved into sterile, pyrogen-free water adjusted to a pH of 7.20, and immediately added to the Hb solution in a molar ratio, Hb/o-ATP 1:3. The reaction is allowed to proceed at 4°C, with 150 rpm stirring, under CO, for 24 hours. Samples of the solution are taken every 6 hours and examined by HPLC, with a size-exclusion column to check the increase in molecular weight of hemoglobin and with an anionic-exchange column to check the change in electrical charge. A Waters HPLC system is used, which comprises a Waters 600 E System Controller, a Waters 712 injector, a Waters 990 Photodiode Detector and a NEC Power Mate 2 computer. The size-exclusion column (Protein Pak 300 SW) and the anionic-exchange column (DEAE-5 PW) are also obtained from Waters (Waters Chromatography Division, Millipore Co., Milford Massachusetts). The ideal crosslinking condition occurs at about 24 hours, when examination by anionic-exchange HPLC reveals 90-95% of o-ATP to have been used in the chemical reaction. As a result, a molecular aggregate is produced that consists of:

| | Molecular weight (kdaltons) | |
|---|---|---|
| 1. Hemoglobin tetramers | 64 | 70% |
| 2. Hemoglobin octamers | 130 | 21% |
| 3. Hemoglobin dodecamers | 195 | 8% |

In other words, under the conditions of the reaction, o-ATP produces mostly intramolecular crosslinking, but also some intermolecular crosslinking. This, however, does not interfere with the following reaction.

After 24 hours, o-adenosine, prepared according to Example IV and stored in powder form, is dissolved into sterile water, pH 7.20 by the addition of Tham (TRADEMARK), with a few drops of ethanol. This compound is added to the Hb solution in a molar ratio of Hb to o-adenosine of 1:5, and the reaction is allowed to continue under the same conditions for 24 hours. At this time, a second dose of o-adenosine is added in the same molar ratio of 1:5 and allowed to react for an additional 24 hours. Samples are examined by HPLC and the chemical reaction is quenched when the level of Hb tetramers has been reduced from 70 to 30%. If the reaction proceeds much beyond this point, polymers of excessive size are produced. GSH dissolved in water + Tham (TRADEMARK), pH 7.20, is then added to the Hb solution in a molar ratio Hb/GSH 1:20, and allowed to react for 16 hours.

At this point, the hemoglobin molecular aggregate includes:

| | | Molecular weight (kdaltons) | |
|---|---|---|---|
| 1. | Hemoglobin tetramer | 64 | 30% |
| 2. | Hemoglobin tetramer x 2 | 130 | 20% |
| 3. | Hemoglobin tetramer x 3 | 195 | 20% |
| 4. | Hemoglobin tetramer x 4- x 6 | 256-390 | 30% |

This aggregate presents a single peak by HPLC-DEAE at 50-51 minutes.

At the end of these chemical reactions, hemoglobin is reconverted from the carboxy-to the oxy- form by repeated flushing with sterilized oxygen at 24608 Kg/Sq meter (35 psi), under gentle stirring at 4°C, plus exposure to 20-second pulses of strong light provided by a "quartzline lamp, DWY, 120 V, 650 W (General Electric Co., Cleveland, Ohio) connected to a "type 4051 molequartz" (Mole-Richardson Co., Hollywood, California). The reoxygenation of hemoglobin can be verified by the use of an IL cooximeter.

E. Preparation of the final product.

In the final step, the Hb solution is dialyzed first against 50mM Tham (Trademark) solution, pH 8.1, then against an electrolyte-balanced saline solution (Normosol R, pH 7.4, containing Sodium 140 mEq/ℓ, Potassium 5 mEq/ℓ, Magnesium 3 mEq/ℓ, Chloride 98 mEq/ℓ, Acetate 27 mEq/ℓ, and Gluconate 23 mEq/ℓ; Abbott Laboratories). The molecular size profile of the final Hb aggregate is:

| | Molecular Weight (daltons) | |
|---|---|---|
| Hb tetramer | 64,000 | 5% |
| Hb tetramer x 2 | 130,000 | 18% |
| Hb tetramer x 3 | 195,000 | 20% |
| Hb tetramer x 4 | 260,000 | 30% |
| Hb tetramer x 5 | 325,000 | 16% |
| Hb tetramer x 6 | 390,000 | 10% |

Thus, the greatest percentage of molecular species is made of four Hb tetramers, with a molecular weight of 260,000 daltons. The aggregate presents a single peak by HPLC-DEAE at 50-51 minutes, which reflects a change in isoelectric point from 6.8 to 6.1.

The dialysate containing discarded hemoglobin can be concentrated to Hb 10 g/dl by dialysis with a 6,000-dalton artificial kidney (90 SCE artifical kidney, C-DAK Co.) and reused for intermolecular crosslinking with o-adenosine.

In its final form, modified hemoglobin is dissolved into Mormosol R, pH 7.4, at the concentration of 10 g/dl. Magnesium chloride ($MgC1_2$), obtained from Matheson Coleman & Bell, Norwood, Ohio, is added to the solution in an amount equimolar with ATP. Mannitol, obtained from GIBCO-Dexter Co., Chagrin Falls, Ohio, is added in a dose of 2 mg/ml of solution.

**EXAMPLE II**

The following procedures were used for the characterization of the new product. Hemoglobin, met-Hb and carboxy-Hb concentrations were measured on a cooximeter (Model 282 Cooximeter, Instrumentation Laboratories, Lexington, Massachusetts). Electrolyte concentrations and osmolarity of the solution were determined by means of an ASTRA apparatus (Beckman Co., Palo Alto, California). Oncotic pressure was assesed by use of a Weil oncometer (Instrumentation Laboratories). Viscosity was determined to 37°C and shear rate of 100/second, by use of a Brookfield viscometer (Brookfield Engineering Laboratories, Stoughton, Massachusetts). The purity of Hb from other proteins, phospholipids and bacterial endotoxins was assessed as described above. Oxygen-binding capacity was calculated from the measurement of Hb concentration and oxygen volume content obtained on the cooximeter. Hb oxygen dissociation curves were obtained on a Hem-O-Scan apparatus (SLM Aminco, American Instruments, Silver Spring, Maryland). $P_{50}$ values were read on these curves under standard conditions of temperature (37°C), pH (7.40) and $pCO_2$ 0.3 Pa (40 torr). Analysis for phosphate content was carried out by the method of Fiske and Subbarow (Journal of Biological Chemistry, Volume 66: pages 375-380, 1925). Determination of GSH content was made according to the method of Reed et al. (Analytical Biochemistry, Volume 106: pages 55-62, 1980). Adenosine was determined by use of HPLC, measuring absorbance at 258 nm, and calculating amount introduced and amount incorporated into hemoglobin. ATP was calculated from the determination of phosphate.

The product here characterized is identified as $(Hb\text{-}PP\text{-}GSH)_n$, where Hb = purified bovine hemoglobin, PP = the two purine derivatives o-ATP and o-adenosine, and GSH = reduced glutathione. The basic molecule is Hb in tetrameric form, shown in Figs. 1 and 2. Its purification from other proteins is illustrated in Fig. 3. For each millimole (mM) of hemoglobin, the compound contains 1.05 mM of ATP, 10 mM of adenosine and 7 mM of GSH. This chemical composition plus HPLC analysis conducted at various intervals during preparation indicate that o-ATP is primarily involved in the intramolecular crosslinking of Hb, while o-adenosine produces the intermollecular crosslinking. In addition, o-adenosine anchors the GSH molecule to Hb. The compound is illustrated in Figs. 4 and 5. Spectrum analysis by HPLC-size exclusion

(Fig. 4) reveals the compound to consist of six molecular species:

| | |
|---|---|
| 1. Hb (tetramer) | = < 5% |
| 2. $(Hb)_2$ | = 18% |
| 3. $(Hb)_3$ | = 20% |
| 4. $(Hb)_4$ | = 30% |
| 5. (Hb) | = 16% |
| 6. $(Hb)_6$ | = 10% |

Among these, $(Hb)_4$, i.e., the aggregate of four tetramers, appears to be the predominant species. Analysis by HPLC-DEAE column (Fig. 5) reveals a single peak at 50-51 minutes, indicating the compound to possess a uniform, reduced (with respect to ummodified Hb) isoelectric point. Analysis by isoelectric focusing (IEF) (Fig. 6) shows these modifications of Hb from another perspective.

Following dialysis with the electrolyte-balanced saline solution, adjusted to a pH of 7.4 by the addition of Tham solution, and following the addition of $MgC1_2$ and mannitol, the final hemoglobin solution has the composition shown in the following table.

At the concentration of 10 g/dl, this Hb solution exposed to atmospheric air transports 13 volumes per cent of oxygen, which indicates an oxygen-binding capacity close to 100% (1.3 volumes of oxygen per 1 g of Hb). The $P_{50}$ volue of the solution is high (~28 mm Hg) despite polymerization due to the high concentration of chloride. The osmolarity is higher than that of plasma, but the viscosity is lower than that of whole blood. The colloid-osmotic pressure is lower than that of plasma despite the high concentration of hemoglobin (compared to albumin), due to the fact that hemoglobin is polymerized, so that the number of "Hb particles" is reduced.

TABLE

| Characteristics of Hemoglobin Solution (Final Product) | |
|---|---|
| 1. Hemoglobin, g/dl | $10.0 \pm 0.5$ |
| 2. Met-Hb, % of hemoglobin | $3.5 \pm 0.5$ |
| 3. Carboxy-Hb, % hemoglobin | $1.5 \pm 0.5$ |
| 4. pH, Units | 7.40 |
| 5. Sodium, mEq/ℓ | $140 \pm 2.0$ |
| 6. Potassium, mEq/ℓ | $4.0 \pm 0.5$ |
| 7. Magnesium, mM/mole of Hb | $0.8 \pm 0.2$ |
| 8. Chloride, mEq/ℓ | $120 \pm 5.0$ |
| 9. Mannitol, mg/dl | $200 \pm 5.0$ |
| 10. Colloid-osmotic pressure, mm Hg | $22 \pm 2.0$ |
| 11. Viscosity, Pa.s ($1.74 \pm 0.04$ cP) | $1.74 \times 10^{-3} \pm 0.04$ |
| 12. Osmolarity, mOsm/ℓ | $325 \pm 10$ |
| 13. Non-Hb proteins | undetectable |
| 14. Stromal phospholipids and lipids | " |
| 15. Bacterial endotoxins | " |
| 16. Sterility | sterile |
| 17. Stability at -60°C | indefinite |

**EXAMPLE III**

Large-scale preparation of o-ATP:

The basic method of preparation of o-ATP is known to the art (see: S.B. Easterbrook-Smith et al., "Pyruvate Carboxylase: Affinity labelling of the magnesium adenosine triphosphate binding site," European Journal of Biochemistry, Volume 62: pages 125-130, 1976. Modifications were made to produce larger quanitites of material and assure a satisfactory chemical reaction with hemoglobin.

Adenosine 5'-triphosphate disodium salt hydrate (ATP), F.W. 551.15, and sodium periodate ($NaIO_4$) 99% purity, F.W. 213.89, were obtained from Aldrich Chemical Company, Milwaukee, Wisconsin. Then 120-ml Sephadex® G-10 columns were obtained from Pharmacia Fine Chemicals, Piscataway, New Jersey. For each column, 550 mg of ATP were dissolved in 15 ml of sterile pyrogen-free water (water for injection, Abbott Laboratories), adjusted with Tham solution to a pH of 7.0, at 0°C. Sodium periodate was added in a molar ratio (ATP/$NaIO_4$) of 1:1.1, and the solution was allowed to stand at 4°C in the dark for one hour. The reaction was stopped by the addition of ethylene glycol in a molar ratio (ATP/ethylene glycol) 2:1 for 15 minutes. The reaction mixture was loaded onto the Sephadex® G-10 column previously equilibrated with "water for injection," at 4°C. The column was eluted with 200 ml of water. The leading half of the nucleotide peak, fractions 20 to 30, as shown in Fig. 7, was pooled and immediately lyophilized with a Labconco Freeze-Dry System with Stoppering Tray Dryer (Labconco Co., Kansas City, Missouri) with vacuum < 10 µm Hg, at -40°C. The powder was stored in dark bottles at -90°C until use.

The concentration of o-ATP is determined by measuring absorbance at 258 nm, while the presence of periodate is assessed by measuring absorbance at 232 nm. The columns are washed with water for injection for 30 hours at 4°C, until the eluate presents less than 0.043 absorbance at 232 nm, i.e., until all periodate has been washed out, before reuse.

Two measures are important for the purpose of this invention: (1) that only fractions containing o-ATP without any trace of periodate be collected; and (2) that these fractions be immediately lyophilized and frozen at -90°C. These measures will prevent the oxidation of hemoglobin upon chemical reaction.

**EXAMPLE IV**

Large-scale preparation of o-adenosine:

The basic method of preparation of o-adenosine is known to the art (see: J.X. Khym and W.E. Cohn, "Characterizations and some chemical reactions of periodate-oxidized nucleotides," Journal of American Chemical Society, Volume 82: pages 6380-6386, 1960). Modifications were made to produce larger quantities of material and to assure a satisfactory checmical reaction with hemoglobin.

Adenosine 98% purity was obtained from Sigma Chemical Co., while sodium periodate was obtained from Aldrich Chemical Co. Adenosine, 6 g, was dissolved in 200 ml of 150 nM $NaIO_4$ in water, at room temperature, for 30 minutes. The solution was passed through a 300 ml column of anion exchange resin AG 1 -X- 8, 100-200 mesh acetate form (Bio-Rad Laboratories, Richmond, California) previously equilibrated with 20 mM acetic acid (Eluent A) obtained from Fisher Scientific Co. The column was eluted with two liters of Eluent A, at the flow rate of 15 ml/minute, temperature 4°C, obtaining fractions of 150 ml. Only fractions 2 to 15 were collected (as shown in Fig. 8), which were immediately lyophilized and frozen, as done for o-ATP.

Before reuse, six liters of 100 mM ammonium chloride (Eluent B) were applied to the column in order to release all periodate. The concentration of periodate in the fractions were determined by measuring absorbance at 232 nm. After this, the column was washed with six liters of "water for injection" and then equilibrated again with 20 mM acetic acid.

Two measures are important for the purpose of this invention: (1) that only fractions containing o-adenosine without any trace of periodate be collected; and (2) that these fractions be immediately lyophilized and frozen at - 90°C. These measures will prevent the oxidation of hemoglobin upon chemical reaction.

**EXAMPLE V**

Toxicity in rabbits: The toxicity of the new composition (Hb-PP-GSH) was tested in rabbits, according to a method previously reported in the scientific literature (M. Feola et al., "Toxicity of polymerized hemoglobin solutions," Surgery, Gynecology and Obstetrics, Volume 166: pages 211-222, 1988).

Twelve New Zealand rabbits of 4.0 kg body weight had sterile cannulae inserted under local anesthesia with 1% lidocaine into the central artery of one ear and the marginal vein of the other ear. The sterile catheter was inserted into the urinary bladder. A thermistor probe and ECG needle-electrodes were inserted under local anesthesia in the limbs. Electrocardiogram (ECG), blood pressure, body temperature, and urinary output were continuously monitored for three hours, after which catheters and electrodes were removed and the animals were returned to their cages. After 30 minutes of steady state (baseline), 80 ml of blood, corresponding to 1/3 of blood volume (calculated blood volume in the rabbit

= 6% of body weight in kg) were removed from the arterial line over a period of 5 minutes. An equal volume of Hb-PP-GSH was infused through the venous line over a period of 30 minutes. This was equal to approximately 2 grams of hemoglobin. The removal of blood caused a drop of blood pressure with an increase in heart rate. These changes were quickly corrected. Moreover, the pulse pressure (difference between systolic and diastolic pressure), which became narrow after the hemorrhage, first returned to normal, then became greater than at baseline, indicating a vasodilator effect of the Hb solution. This effect lasted the entire acute period of observation of three hours. The ECG showed no cardiac arrhythmia. The urinary output remained normal without any extravasation of hemoglobin into the urine.

Blood samples taken 30 minutes, 1, 3 and 24 hours after blood replacement revealed: (1) no reduction of white blood cells and platelets in excess of the hemodiluting effect; (2) no activation of intravascular coagulation and fibrinolysis, as determined by measurement of serum fibrinogen, prothrombin time and fibrin split products; (3) no elevation of creatine phosphokinase brain isoenzyme (CPK-BB) or myocardial isoenzyme (CPK-MB) that would suggest cerebral or myocardial damage; (4) no elevation of serum glutamic pyruvic transaminase (SGPT) suggestive of liver injury; (5) normal arterial blood gases indicative of normal pulmonary function; and (6) normal serum creatinine suggestive of normal renal function. Combined blood and urine samples at 3 and 24 hours revealed normal creatinine clearance, again indicative of normal renal function. Whole blood oxygen dissociation curves showed no change in $P_{50}$ value, i.e., no increase in oxygen affinity due to hemoglobin. The level of plasma Hb at 24 hours was approximately 50% of the initial level, suggesting a hemoglobin half-life of 24 hours.

The animals appeared and behaved normally for 24 hours. At this time, they were killed and the vital organs were examined histologically. None of the pathological changes previously reported in the scientific literature were found (a) heart, (b) lungs, (c) liver and (d) kidneys. These findings contrast sharply with those previously reported (see reference above) following the use of non-pure hemoglobin crosslinked with glutaraldehyde.

**EXAMPLE VI**

Efficacy in rabbits: The efficacy of the product as a blood substitute was tested in rabbits. Following instrumentation similar to that described in the previous example, a control group of 10 New Zealand rabbits of 4.0 kg body weight were subjected to the removal of 1/3 of calculated blood volume (blood volume = 6% of body weight in kg), followed by the removal of another 1/3 after 15 minutes. Without treatment, all of these animals died within one hour. An experimental group of 10 rabits was subjected to the same procedure, but received an infusion of Hb-PP-GSH in the same volume as the total blood loss. All of these animals survived and reconstituted their baseline hematocrit (concentration of red blood cells) within seven days.

**EXAMPLE VII**

Vasodilation after blood replacement in rats: Twelve Sprague-Dawley rats weighing 350-450 g were anesthetized by intraperitoneal injection of sodium pentobarbital, 45 mg/kg, and placed on a surgical board in the supine position. The right femoral artery, carotid artery and external jugular vein were surgically exposed and cannulated with polyethylene catheters (model PE 50; Intramedic, New York). The external jugular catheter was advanced into the right atrium, while a thermistor probe (model IF, Columbus Instruments, Columbus, Ohio) was advanced through the carotid artery into the ascending aorta. Each of the catheters was filled with saline solution containing bovine heparin 5 IU/ml. The femoral arterial and the jugular venous lines were connected to pressure transducers. Needle electrodes were inserted subcutaneously into the limbs and used to monitor the electrocardiogram (ECG). Heating lamps were adjusted to maintain constant body temperature.

Heart rate was determined from the ECG tracing, cardiac output was measured by thermodilution, by injecting 200 µl of saline solution maintained at room temperature (20-22°C) into the right atrium and recording a thermodilution curve from the aortic thermistor. Systemic vascular resistance was calculated as the mean arterial pressure minus the right atrial pressure divided by the cardiac output.

Following recording of baseline hemodynamic data, 1/3 of calculated blood volume (blood volume in the rate = 7% of body weight in Kg) was removed from the arterial line over a 5-minute period. After 15 minutes, Hb-PP-GSH in the same volume was infused through the venous line. Heart rate, mean arterial pressure, and cardiac output were measured and systemic vascular resistance was calculated at baseline ($T_1$), 15 minutes after blood removal ($T_2$), and 15 minutes after hemoglobin infusion ($T_3$). Statistical analysis of the data was carried out using Student's t-test for paired data.

The results, summarized below, show increased systemic vascular resistance after blood removal, followed by reduction to normal and by vasodilation, even with respect to baseline, after bood replacement.

TABLE

| Hemodynamic profiles after blood replacement with Hb-PP-GSH | | | |
|---|---|---|---|
| | Baseline | Hemorrhage | Hemoglobin |
| Heart Rate (beats/minute) | 320 ± 5 | 390 ± 10* | 300 ± 10* |
| Mean Arterial Pressure (mm Hg) | 105 ± 5 | 90 ± 3* | 105 ± 5* |
| Cardiac Output (ml/kg/minute) | 425 ± 20 | 275 ± 15* | 455 ± 28* |
| Systemic Vascular Resistance (mm Hg/ml/kg/minutes | 0.23±0.02 | 0.33±0 .03* | 0.21±0.02* |
| Numbers = Mean Values ± Standard Deviation; * = statistically significant difference (P < 0.05) from previous time interval. | | | |

**EXAMPLE VII**

Generation of oxygen free-radicals in rabbits:

Twelve New Zealand rabbits of 4.0 kg body weight were sedated with chlorpromazine (5 mg/kg, intramuscularly) and subjected to limited instrumentation. Sterile plastic cannulae were inserted into the central artery and the marginal vein of one ear, and a thermistor probe and needle electrodes were inserted subcutaneously into the limbs. One-third of calculated blood volume (2% of body weight in kg) was removed from the arterial line over a period of five minutes and the same volume of Hb solution was infused through the vein over a period of 30 minutes. One control group of six rabbits received unmodified Hb, while the experimental group (six rabbits) received Hb-PP-GSH. The effects were studied in terms of plasma levels of hydrogen perioxide ($H_20_2$) and lipid peroxides, determined at baseline, and 15 minutes, 1 hour, 3 hours and 24 hours after Hb infusion. Plasma Hb and met-Hb were also measured at the same time intervals.

$H_20_2$ increased in the group receiving unmodified Hb from 2 + 2 to 70 + 5 micromoles/milliliter after one hour, then decreased to 50 + 5 μmol/ml at three hours and to 10 + 5 μmol/ml at one hour, and returned to baseline after three hours. Similarly, lipid peroxides increased from 1.5 + 0.9 nanomoles/milliliter at baseline to 4.0 + 1.0 nmol/ml after one hour in the control group. No significant increase occurred in the experimental group. Plasma met-Hb increased from 0 to 15% in one hour in the group that received unmodified Hb. It increased from 0 to 5% in the group that received Hb-PP-GSH. The difference in these variables between the two groups was found signfiicant by statistical analysis, Student's t-tests for unpaired and paired samples plus ANOVA.

**Claims**

**Claims for the following Contracting States : AT, BE, CH, DE, DK, FR, GB, GR, IT, LI, LU, NL, SE**

1. A composition for use as a blood substitute comprising a hemoglobin reacted with o-ATP and o-adenosine to form a crosslinked product, and reacted with a quantity of reduced glutathione, the product being dissolved in a saline solution.

2. The composition according to claim 1 further comprising magnesium chloride at a concentration approximately equimolar with the o-ATP.

3. The composition according to claim 1 wherein the hemoglobin is crosslinked intramolecularly with periodate-oxidized ATP and intermolecularly with periodate-oxidized adenosine to form molecules of polyhemoglobin.

4. The composition according to claim 1 wherein the hemoglobin, o-ATP, o-adenosine, and glutathione are present in approximate molar ratios of 1:1.05:10:7, respectively.

5. The composition according to claim 1 wherein the crosslinked hemoglobin molecules have molecular weights ranging from approximately 130 to approximately 390 kilodaltons.

6. The composition according to claim 1 wherein less than 5% of the hemoglobin is oxidized to met-hemoglobin.

7. The composition according to claim 1 wherein the hemoglobin is of bovine origin.

8. A method for extracting purified hemoglobin from whole blood, comprising the steps of:

centrifugation of the whole blood to remove platelets and plasma and suspending the resulting leukocyte-erythrocyte mixture in solution;

cooling the leukocyte-erythrocyte mixture to aggregate the leukocytes;

removing the leukocyte aggregate by filtration;

extracting hemoglobin from the erythrocytes by dialyzing against a hypotonic solution, and subjecting the erythrocytes to ultrafiltration under increased hydrostatic pressure;

converting the hemoglobin to carboxy form by saturating the hemoglobin with carbon monoxide;

pasteurizing the hemoglobin solution to denature and precipitate non-heme proteins;

centrifuging the hemoglobin solution mixed with chloroform to remove phospholipids and precipitated non-heme proteins;

removing any remaining chloroform from the supernatant hemoglobin solution by flushing with carbon monoxide gas; and

removing endotoxins from the hemoglobin solution by passing the solution through an affinity chromatography column.

9. A method for preparing a composition according to claim 1 suitable for use as a blood substitute, comprising the steps of:

converting a solution of purified hemoglobin to the carboxy form by saturating the hemoglobin with carbon monoxide;

dialyzing the resulting carboxy-hemoglobin solution against a normotonic solution to concentrate the hemoglobin to approximately 10 g/dl;

reacting the carboxy-hemoglobin with oxidized adenosine triphosphate (o-ATP) to effect predominantly intramolecular crosslinking among the hemoglobin molecules;

reacting the carboxy-hemoglobin with oxidized adenosine (o-adenosine) to effect predominantly intermolecular crosslinking between the hemoglobin molecules;

quenching the o-adenosine crosslinking reaction by adding reduced glutathione to the solution; and

converting the hemoglobin from the carboxy- to the oxy- form by flushing the solution with oxygen.

10. The method according to claim 9 further comprising the step of dissolving the hemoglobin composition in a solution containing magnesium chloride.

11. The method according to claim 10 further comprising the step of adding mannitol to the composition.

12. The method according to claim 9 wherein the o-adenosine and o-ATP are prepared by periodate oxidation of adenosine and ATP, respectively, the periodate being removed prior to reacting the o-ATP and adenosine with hemoglobin.

13. The method according to claim 11 wherein the purified hemoglobin is obtained from whole blood using the method as set forth in claim 8.

14. The method according to claim 13 wherein the whole blood is of bovine origin.

15. The hemoglobin composition obtainable by the method set forth in claim 14.

16. The use of a composition according to any of claims 1 to 7 for the preparation of a medicament for administration to an animal in need of therapy.

17. The use of a composition according to any of claims 1 to 7 for the preparation of an emergency resuscitative fluid for administration to an animal suffering from blood loss.

**Claims for the following Contracting State : ES**

1. A method for making a composition for use as a blood substitute comprising reacting a hemoglobin with o-ATP and o-adenosine to effect crosslinking, reacted with a quantity of reduced glutathione, and dissolving in a saline solution.

2. The method according to claim 1 further comprising adding magnesium chloride at a concentration approximately equimolar with the o-ATP.

3. The method according to claim 1 wherein the hemoglobin is crosslinked intramolecularly by reacting with periodate-oxidized ATP and intermolecularly by reacting with periodate-oxidized adenosine.

4. The method according to claim 1 wherein the hemoglobin, o-ATP, o-adenosine, and glutathione are added in approximate molar ratios of 1:1.05:10:7 respectively.

5. The method according to claim 1 wherein the crosslinked hemoglobin molecules have molecular weights ranging from approximately 130 to approximately 390 kilodaltons.

6. The method according to claim 1 wherein less than 5% of the hemoglobin is oxidized to met-hemoglobin.

7. The method according to claim 1 wherein the hemoglobin is of bovine origin.

8. A method for extracting purified hemoglobin from whole blood, comprising the steps of:

centrifugation of the whole blood to remove platelets and plasma and suspending the resulting leukocyte-erythrocyte mixture in solution;

cooling the leukocyte-erythrocyte mixture to aggregate the leukocytes;

removing the leukocyte aggregate by filtration;

extracting hemoglobin from the erythrocytes by dialyzing against a hypotonic solution, and subjecting the erythrocytes to ultrafiltration under increased hydrostatic pressure;

converting the hemoglobin to carboxy form by saturating the hemoglobin with carbon monoxide;

pasteurizing the hemoglobin solution to denature and precipitate non-heme proteins;

centrifuging the hemoglobin solution mixed with chloroform to remove phospholipids and precipitated non-heme proteins;

removing any remaining chloroform from the supernatant hemoglobin solution by flushing with carbon monoxide gas; and

removing endotoxins from the hemoglobin solution by passing the solution through an affinity chromatography column.

9. A method for preparing a composition according to claim 1 suitable for use as a blood substitute, comprising the steps of:

converting a solution of purified hemoglobin to the carboxy form by saturating the hemoglobin with carbon monoxide;

dialyzing the resulting carboxy-hemoglobin solution against a normotonic solution to concentrate the hemoglobin to approximately 10 g/dl;

reacting the carboxy-hemoglobin with oxidized adenosine triphosphate (o-ATP) to effect predominantly intramolecular crosslinking among the hemoglobin molecules;

reacting the carboxy-hemoglobin with oxidized adenosine (o-adenosine) to effect predominantly intermolecular crosslinking between the hemoglobin molecules;

quenching the o-adenosine crosslinking reaction by adding reduced glutathione to the solution; and

converting the hemoglobin from the carboxy- to the oxy- form by flushing the solution with oxygen.

10. The method according to claim 9 further comprising the step of dissolving the hemoglobin composition in a solution containing magnesium chloride.

11. The method according to claim 10 further comprising the step of adding mannitol to the composition.

12. The method according to claim 9 wherein the o-adenosine and o-ATP are prepared by periodate oxidation of adenosine and ATP, respectively, the periodate being removed prior to reacting the o-ATP and adenosine with hemoglobin.

13. The method according to claim 11 wherein the purified hemoglobin is obtained from whole blood using the method as set forth in claim 6.

14. The method according to claim 13 wherein the whole blood is of bovine origin.

15. The use of a composition obtainable by the method according to any of claims 1 to 7 or 9 to 14 for the preparation of a medicament for administration to an animal in need of therapy.

16. The use of a composition obtainable by the method according to any of claims 1 to 7 or 9 to 14 for the preparation of an emergency resuscitative fluid for administration to an animal suffering from blood loss.

**Patentansprüche**

**Patentansprüche für folgende Vertragsstaaten : AT, BE, CH, DE, DK, FR, GB, GR, IT, LI, LU, NL, SE**

1. Zusammensetzung zur Verwendung als ein Blutersatzstoff, umfassend Hämoglobin, umgesetzt mit oxidiertem Adenosintriphosphat (o-ATP) und oxidiertem Adenosin (o-Adenosin) unter Bildung eines vernetzten Produkts, sowie umgesetzt mit einer Menge von reduziertem Glutathion, wobei das Produkt in einer Kochsalzlösung aufgelöst ist.

2. Zusammensetzung nach Anspruch 1, ferner umfassend Magnesiumchlorid in einer Konzentration von näherungsweise äquimolar mit dem o-ATP.

3. Zusammensetzung nach Anspruch 1, bei welcher das Hämoglobin intramolekular mit Periodat-oxidiertem ATP sowie intermolekular mit Periodat-oxidiertem Adenosin vernetzt ist, um Polyhämoglobin-Moleküle zu bilden.

4. Zusammensetzung nach Anspruch 1, bei welcher das Hämoglobin, o-ATP, o-Adenosin bzw. Glutathion in näherungsweise molaren Anteilen von 1:1,05:10:7 vorliegen.

5. Zusammensetzung nach Anspruch 1, bei welcher die vernetzten Hämoglobin-Moleküle relative Molekülmassen im Bereich von näherungsweise 130 bis näherungsweise 390 k-Dalton haben.

6. Zusammensetzung nach Anspruch 1, bei welcher weniger als 5% des Hämoglobins zu MET-Hämoglobin oxidiert sind.

7. Zusammensetzung nach Anspruch 1, bei welcher das Hämoglobin bovinen Ursprungs ist.

8. Verfahren zum Extrahieren von gereinigtem Hämoglobin aus Vollblut, umfassend die Schritte:
Zentrifugieren des Vollbluts zur Entfernung von Blutplättchen und Plasma und Suspendieren der resultierenden Leukocyten/Erythrocyten-Mischung in Lösung;
Kühlen der Leukocyten/Erythrocyten-Mischung zum Aggregiern der Leukocyten;
Entfernen des Leukocyten-Aggregats durch Filtration;
Extrahieren von Hämoglobin aus den Erythrocyten durch Dialysieren gegen eine hypotonische Lösung und Ultrafiltrieren der Erythrocyten unter erhöhtem hydrostatischem Druck;
Umwandeln des Hämoglobins in seine Carboxy-Form durch Sättigen des Hämoglobins mit Kohlenmonoxid;
Pasteurisieren der Hämoglobin-Lösung, um Nicht-Hämoproteine zu denaturieren und auszufällen;
Zentrifugieren der mit Chloroform gemischten Hämoglobin-Lösung, um Phospholipide und ausgefällte Nicht-Hämoproteine zu entfernen;
Entfernen von sämtlichen verbleibenden Chloroform aus der Überstand Hämoglobin-Lösung durch Spülen mit Kohlenmonoxid-Gas; sowie
Entfernen der Endotoxine aus der Hämoglobin-Lösung, indem die Lösung durch eine Affinitätschromatographie-Säule geschickt wird.

9. Verfahren zum Herstellen einer zur Verwendung als ein Blutersatzstoff geeigneten Zusammensetzung nach Anspruch 1, umfassend die Schritte:
Umwandeln einer Lösung aus gereinigtem Hämoglobin in die Carboxy-Form durch Sättigen des Hämoglobins mit Kohlenmonoxid;
Dialysieren der resultierenden Carboxy-Hämoglobin-Lösung gegen eine normotonische Lösung zum Konzentrieren des Hämoglobins auf etwa 10 g/dl;
Umsetzen des Carboxy-Hämoglobins mit oxidiertem Adenosintriphosphat (o-ATP), um ein überwiegend intramolekulares Vernetzen zwischen den Hämoglobin-Molekülen herbeizuführen;
Umsetzen des Carboxy-Hämoglobins mit oxidiertem Adenosin (o-Adenosin), um ein überwiegend intermolekulares Vernetzen zwischen den Hämoglobin-Molekülen herbeizuführen;
Quenchen der o-Adenosin-Vernetzungsreaktion durch Zusetzen von reduziertem Glutathion zur Lösung;

sowie
Umwandeln des Hämoglobins von der Carboxy- in die Oxy-Form durch Spülen der Lösung mit Sauerstoff.

10. Verfahren nach Anspruch 9, ferner umfassend den Schritt des Auflösens der Hämoglobin-Zusammensetzung in einer Magnesiumchlorid enthaltenden Lösung.

11. Verfahren nach Anspruch 10, ferner umfassend den Schritt des Zusetzens von Mannitol zu der Zusammensetzung.

12. Verfahren nach Anspruch 9, bei welchem das o-Adenosin und o-ATP hergestellt werden durch Periodat-Oxidation von Adenosin bzw. ATP, wobei das Periodat vor dem Umsetzen des o-ATP und Adenosins mit Hämoglobin entfernt wird.

13. Verfahren nach Anspruch 11, bei welchem das gereinigte Hämoglobin unter Anwendung des Verfahren nach Anspruch 8 aus Vollblut erhalten wird.

14. Verfahren nach Anspruch 13, bei welchem das Vollblut bovinen Ursprungs ist.

15. Hämoglubin-Zusammensetzung, die nach dem Verfahren nach Anspruch 14 erhalten werden kann.

16. Verwendung einer Zusammensetzung zur Herstellung eines Medikaments zur Verabreichung an einem Tier, welches einer Therapie bedarf, welche Zusammensetzung nach einem Verfahren gemäß einem der Ansprüche 1 bis 7 erhalten werden kann.

17. Verwendung einer Zusammensetzung zur Herstellung eines Notfall-Reanimationsfluids für die Verabreichung an einem Tier, das starken Blutverlust erleidet, welche Zusammensetzung nach einem Verfahren gemäß einem der Ansprüche 1 bis 7 erhalten werden kann.

**Patentansprüche für folgenden Vertragsstaat : ES**

1. Verfahren zur Herstellung einer Zusammensetzung zur Verwendung als ein Blutersatzstoff durch Umsetzen eines Hämoglobins mit oxidiertem Adenosintriphosphat (o-ATP) und oxidiertem Adenosin (o-Adenosin), um ein Vernetzen herbeizuführen, Umsetzen mit einer Menge von reduziertem Glutathion, und Auflösen in einer Kochsalzlösung.

2. Verfahren nach Anspruch 1, ferner umfassend Zusetzen von Magnesiumchlorid in einer Konzentration von näherungsweise äquimolar mit dem o-ATP.

3. Verfahren nach Anspruch 1, bei welchem das Hämoglobin intramolekular durch Umsetzen mit Periodat-oxidiertem ATP, sowie intermolekular durch Umsetzen mit Periodat-oxidiertem Adenosin vernetzt ist.

4. Verfahren nach Anspruch 1, bei welchem das Hämoglobin, o-ATP, o-Adenosin bzw. Glutathion in näherungsweise molaren Anteilen von 1:1,05:10:7 zugesetzt werden.

5. Verfahren nach Anspruch 1, bei welchem die vernetzten Hämoglobin-Moleküle relative Molekülmassen im Bereich von näherungsweise 130 bis näherungsweise 390 k-Dalton haben.

6. Verfahren nach Anspruch 1, bei welchem weniger als 5% des Hämoglobins zu MET-Hämoglobin oxidiert sind.

7. Verfahren nach Anspruch 1, bei welchem das Hämoglobin bovinen Ursprungs ist.

8. Verfahren zum Extrahieren von gereinigtem Hämoglobin aus Vollblut, umfassend die Schritte:
Zentrifugieren des Vollbluts zur Entfernung von Blutplättchen und Plasma und Suspendieren der resultierenden Leukocyten/Erythrocyten-Mischung in Lösung;
Kühlen der Leukocyten/Erythrocyten-Mischung zum Aggregiern der Leukocyten;
Entfernen des Leukocyten-Aggregats durch Filtration;
Extrahieren von Hämoglobin aus den Erythrocyten durch Dialysieren gegen eine hypotonische Lösung und Ultrafiltrieren der Erythrocyten unter erhöhtem hydrostatischem Druck;
Umwandeln des Hämoglobins in seine Carboxy-Form durch Sättigen des Hämoglobins mit Kohlenmonoxid;
Pasteurisieren der Hämoglobin-Lösung, um Nicht-Hämoproteine zu denaturieren und auszufällen;
Zentrifugieren der mit Chloroform gemischten Hämoglobin-Lösung, um Phospholipide und ausgefällte Nicht-Hämoproteine zu entfernen;
Entfernen von sämtlichen verbleibenden Chloroform aus der Überstand Hämoglobin-Lösung durch Spülen mit Kohlenmonoxid-Gas; sowie
Entfernen der Endotoxine aus der Hämoglobin-Lösung, indem die Lösung durch eine Affinitätschromatographie-Säule geschickt wird.

**9.** Verfahren zum Herstellen einer zur Verwendung als ein Blutersatzstoff geeigneten Zusammensetzung nach Anspruch 1, umfassend die Schritte:
Umwandeln einer Lösung aus gereinigtem Hämoglobin in die Carboxy-Form durch Sättigen des Hämoglobins mit Kohlenmonoxid;
Dialysieren der resultierenden Carboxy-Hämoglobin-Lösung gegen eine normotonische Lösung zum Konzentrieren des Hämoglobins auf etwa 10 g/dl;
Umsetzen des Carboxy-Hämoglobins mit oxidiertem Adenosintriphosphat (o-ATP), um ein überwiegend intramolekulares Vernetzen zwischen den Hämoglobin-Molekülen herbeizuführen;
Umsetzen des Carboxy-Hämoglobins mit oxidiertem Adenosin (o-Adenosin), um ein überwiegend intermolekulares Vernetzen zwischen den Hämoglobin-Molekülen herbeizuführen;
Quenchen der o-Adenosin-Vernetzungsreaktion durch Zusetzen von reduziertem Glutathion zur Lösung; sowie
Umwandeln des Hämoglobins von der Carboxy- in die Oxy-Form durch Spülen der Lösung mit Sauerstoff.

**10.** Verfahren nach Anspruch 9, ferner umfassend den Schritt des Auflösens der Hämoglobin-Zusammensetzung in einer Magnesiumchlorid enthaltenden Lösung.

**11.** Verfahren nach Anspruch 10, ferner umfassend den Schritt des Zusetzens von Mannitol zu der Zusammensetzung,

**12.** Verfahren nach Anspruch 9, bei welchem das o-Adenosin und o-ATP hergestellt werden durch Periodat-Oxidation von Adenosin bzw. ATP, wobei das Periodat vor dem Umsetzen des o-ATP und Adenosins mit Hämoglobin entfernt wird.

**13.** Verfahren nach Anspruch 11, bei welchem das gereinigte Hämoglobin unter Anwendung des Verfahren nach Anspruch 6 aus Vollblut erhalten wird.

**14.** Verfahren nach Anspruch 13, bei welchem das Vollblut bovinen Ursprungs ist.

**15.** Verwendung einer Zusammensetzung zur Herstellung eines Medikaments für die Verabreichung an einem Tier, welches einer Therapie bedarf, welche Zusammensetzung nach einem Verfahren gemäß einem der Ansprüche 1 bis 7 oder 9 bis 14 erhalten werden kann.

**16.** Verwendung einer Zusammensetzung zur Herstellung eines Notfall-Reanimationsfluids für die Verabreichung an einem Tier, das starken Blutverlust erleidet, welche Zusammensetzung nach einem Verfahren gemäß einem der Ansprüche 1 bis 7 oder 9 bis 14 erhalten werden kann.

**Revendications**

**Revendications pour les Etats contractants suivants : AT, BE, CH, DE, DK, FR, GB, GR, IT, LI, LU, NL, SE**

**1.** Composition pour une utilisation en tant que substitut sanguin comprenant une hémoglobine qui a réagi avec de l'o-ATP et de l'o-adénosine pour former un produit réticulé, et qui a réagi avec une quantité de glutathion réduit, le produit étant dissous dans une solution saline.

**2.** Composition selon la revendication 1 comprenant de plus du chlorure de magnésium à une concentration approximativement équimolaire à l'o-ATP.

**3.** Composition selon la revendication 1 dans laquelle l'hémoglobine est réticulée de façon intramoléculaire avec de l'ATP oxydée au periodate et de façon intermoléculaire avec de l'adénosine oxydée au periodate pour former des molécules de polyhémoglobine.

**4.** Composition selon la revendication 1 dans laquelle l'hémoglobine, l'o-ATP, l'o-adénosine et le glutathion sont présents dans des rapports molaires approximatifs de 1:1.05:10:7, respectivement.

5. Composition selon la revendication 1 dans laquelle les molécules d'hémoglobine réticulée ont des poids moléculaires s'étendant de approximativement 130 à approximativement 390 kilodaltons.

6. Composition selon la revendication 1 dans laquelle moins de 5% de l'hémoglobine est oxydée en met-hémoglobine.

7. Composition selon la revendication 1 dans laquelle l'hémoglobine est d'origine bovine.

8. Procédé d'extraction d'hémoglobine purifiée du sang complet, comprenant les étapes de:
centrifuger du sang complet pour éliminer les plaquettes et le plasma et mettre en suspension le mélange leucocyte-érythrocyte résultant en solution;
refroidir le mélange leucocyte-érythrocyte pour agréger les leucocytes;
éliminer l'agrégat de leucocytes par filtration;
extraire l'hémoglobine des érythrocytes en dialysant contre une solution hypotonique, et soumettre les érythrocytes à une ultrafiltration sous pression hydrostatique accrue;
convertir l'hémoglobine en sa forme carboxy en saturant l'hémoglobine avec du monoxyde de carbone;
pasteuriser la solution d'hémoglobine pour dénaturer et précipiter les protéines dépourvues d'hème;
centrifuger la solution d'hémoglobine mélangée avec du chloroforme pour éliminer les phospholipides et les protéines dépourvues d'hème précipitées;
éliminer tout chloroforme restant de la solution d'hémoglobine surnageante en purgeant avec du monoxyde de carbone; et
éliminer les endotoxines de la solution d'hémoglobine en faisant passer la solution au travers d'une colonne de chromatographie d'affinité.

9. Procédé de préparation d'une composition selon la revendication 1 adaptée à un usage en tant que substitut sanguin, comprenant les étapes de:
convertir une solution d'hémoglobine purifiée en sa forme carboxy en saturant l'hémoglobine avec du monoxyde de carbone;
dialyser la solution de carboxy-hémoglobine résultante contre une solution normotonique pour concentrer l'hémoglobine à approximativement 10 g/dl;
faire réagir la carboxy-hémoglobine avec de l'adénosine triphosphate oxydée (o-ATP) pour effectuer d'une manière prédominante une réticulation intramoléculaire parmi les molécules d'hémoglobine;
faire réagir la carboxy-hémoglobine avec de l'adénosine oxydée (o-adénosine) pour effectuer d'une manière prédominante une réticulation intermoléculaire entre les molécules d'hémoglobine;
arrêter la réaction de réticulation par l'o-adénosine en ajoutant du glutathion réduit à la solution; et
convertir l'hémoglobine de sa forme carboxy à sa forme oxy en purgeant la solution avec de l'oxygène.

10. Procédé selon la revendication 9 comprenant de plus l'étape de dissoudre la composition à base d'hémoglobine dans une solution contenant du chlorure de magnésium.

11. Procédé selon la revendication 10 comprenant de plus l'étape d'ajouter du mannitol à la composition.

12. Procédé selon la revendication 9 dans lequel l'o-adénosine et l'o-ATP sont préparées par une oxydation d'adénosine et d'ATP par du periodate, respectivement, le periodate étant éliminé avant de faire réagir l'o-ATP et l'adénosine avec de l'hémoglobine.

13. Procédé selon la revendication 11 dans lequel l'hémoglobine purifiée est obtenue à partir de sang complet en utilisant le procédé tel que décrit dans la revendication 8.

14. Procédé selon la revendication 13 dans lequel le sang complet est d'origine bovine.

15. Composition à base d'hémoglobine qu'il est possible d'obtenir par le procédé décrit dans la revendication 14.

16. Utilisation d'une composition selon l'une quelconque des revendications 1 à 7 pour la préparation d'un médicament pour l'administration à un animal nécessitant une thérapie.

17. Utilisation d'une composition selon l'une quelconque des revendications 1 à 7 pour la préparation d'un fluide de réanimation d'urgence pour l'administration à un animal souffrant d'une perte de sang.

**Revendications pour l'Etat contractant suivant : ES**

1. Procédé pour préparer une composition pour une utilisation en tant que substitut sanguin comprenant la réaction d'une hémoglobine avec de l'o-ATP et de l'o-adénosine pour effectuer une réticulation, la réaction avec une quantité de glutathion réduit, et la dissolution dans une solution saline.

2. Procédé selon la revendication 1 comprenant de plus l'ajout de chlorure de magnésium à une concentration approximativement équimolaire à l'o-ATP.

3. Procédé selon la revendication 1 dans lequel l'hémoglobine est réticulée de façon intramoléculaire par réaction avec de l'ATP oxydé au periodate et de façon intermoléculaire par réaction avec de l'adénosine oxydée au periodate.

4. Procédé selon la revendication 1 dans lequel l'hémoglobine, l'o-ATP, l'o-adénosine et le glutathion sont ajoutés dans des rapports molaires approximatifs de 1:1,05:10:7, respectivement.

5. Procédé selon la revendication 1 dans lequel les molécules d'hémoglobine réticulée ont des poids moléculaires s'étendant de approximativement 130 à approximativement 390 kilodaltons.

6. Procédé selon la revendication 1 dans lequel moins de 5% de l'hémoglobine est oxydée en met-hémoglobine.

7. Procédé selon la revendication 1 dans lequel l'hémoglobine est d'origine bovine.

8. Procédé d'extraction d'hémoglobine purifiée du sang complet, comprenant les étapes de:
   centrifuger du sang complet pour éliminer les plaquettes et le plasma et mettre en suspension le mélange leucocyte-érythrocyte résultant en solution;
   refroidir le mélange leucocyte-érythrocyte pour agréger les leucocytes;
   éliminer l'agrégat de leucocytes par filtration;
   extraire l'hémoglobine des érythrocytes en dialysant contre une solution hypotonique, et soumettre les érythrocytes à une ultrafiltration sous pression hydrostatique accrue;
   convertir l'hémoglobine en sa forme carboxy en saturant l'hémoglobine avec du monoxyde de carbone;
   pasteuriser la solution d'hémoglobine pour dénaturer et précipiter les protéines dépourvues d'hème;
   centrifuger la solution d'hémoglobine mélangée avec du chloroforme pour éliminer les phospholipides et les protéines dépourvues d'hème précipitées;
   éliminer tout chloroforme restant de la solution d'hémoglobine surnageante en purgeant avec du monoxyde de carbone; et
   éliminer les endotoxines de la solution d'hémoglobine en faisant passer la solution au travers d'une colonne de chromatographie d'affinité.

9. Procédé de préparation d'une composition selon la revendication 1 adaptée à un usage en tant que substitut sanguin, comprenant les étapes de:
   convertir une solution d'hémoglobine purifiée en sa forme carboxy en saturant l'hémoglobine avec du monoxyde de carbone;
   dialyser la solution de carboxy-hémoglobine résultante contre une solution normotonique pour concentrer l'hémoglobine à approximativement 10 g/dl;
   faire réagir la carboxy-hémoglobine avec de l'adénosine triphosphate oxydée (o-ATP) pour effectuer d'une manière prédominante une réticulation intramoléculaire parmi les molécules d'hémoglobine;
   faire réagir la carboxy-hémoglobine avec de l'adénosine oxydée (o-adénosine) pour effectuer d'une manière prédominante une réticulation intermoléculaire entre les molécules d'hémoglobine;
   arrêter la réaction de réticulation par l'o-adénosine en ajoutant du glutathion réduit à la solution; et
   convertir l'hémoglobine de sa forme carboxy à sa forme oxy en purgeant la solution avec de l'oxygène.

10. Procédé selon la revendication 9 comprenant de plus l'étape de dissoudre la composition à base d'hémoglobine dans une solution contenant du chlorure de magnésium.

11. Procédé selon la revendication 10 comprenant de plus l'étape d'ajouter du mannitol à la composition.

12. Procédé selon la revendication 9 dans lequel l'o-adénosine et l'o-ATP sont préparées par une oxydation d'adénosine et d'ATP par du periodate, respectivement, le periodate étant éliminé avant de faire réagir l'o-ATP et l'adénosine avec de l'hémoglobine.

**13.** Procédé selon la revendication 11 dans lequel l'hémoglobine purifiée est obtenue à partir de sang complet en utilisant le procédé tel que décrit dans la revendication 6.

**14.** Procédé selon la revendication 13 dans lequel le sang complet est d'origine bovine.

**15.** Utilisation d'une composition qu'il est possible d'obtenir par le procédé selon l'une quelconque des revendications 1 à 7 ou 9 à 14 pour la préparation d'un médicament pour l'administration à un animal nécessitant une thérapie.

**16.** Utilisation d'une composition qu'il est possible d'obtenir par le procédé selon l'une quelconque des revendications 1 à 7 ou 9 à 14 pour la préparation d'un fluide de réanimation d'urgence pour l'administration à un animal souffrant d'une perte de sang.

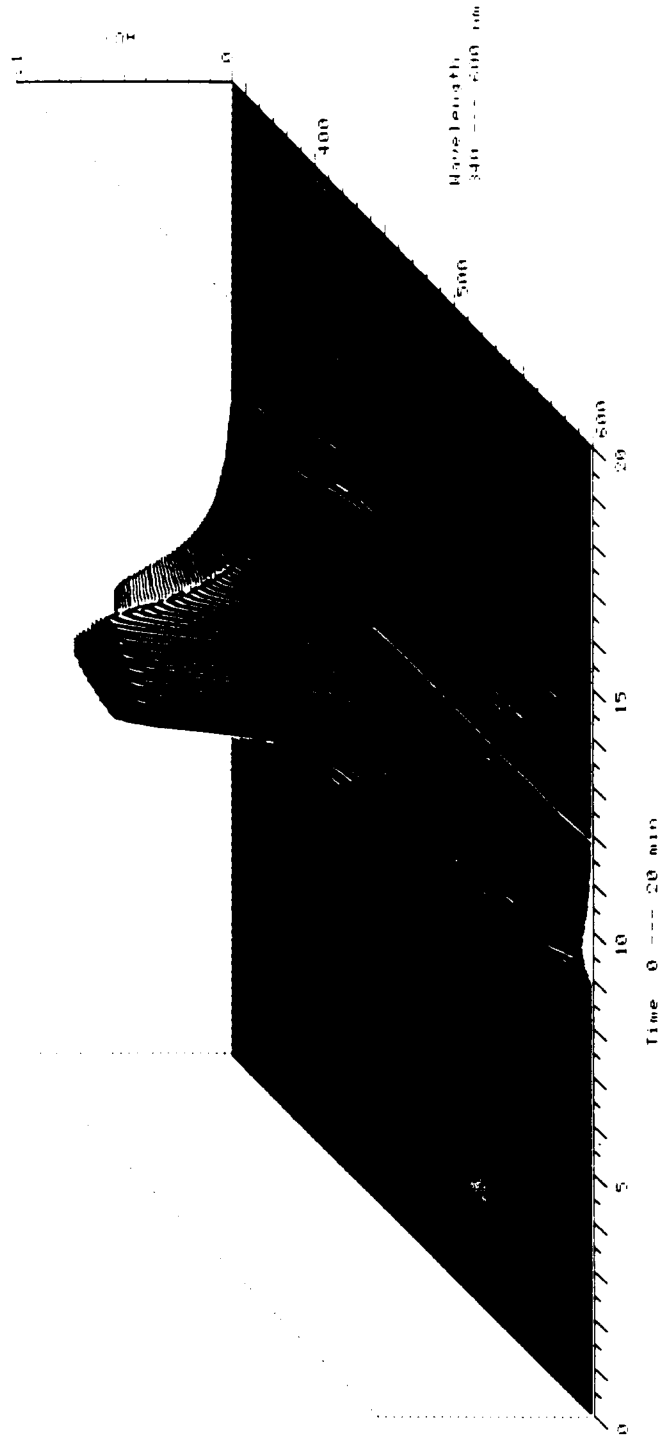


FIGURE 1

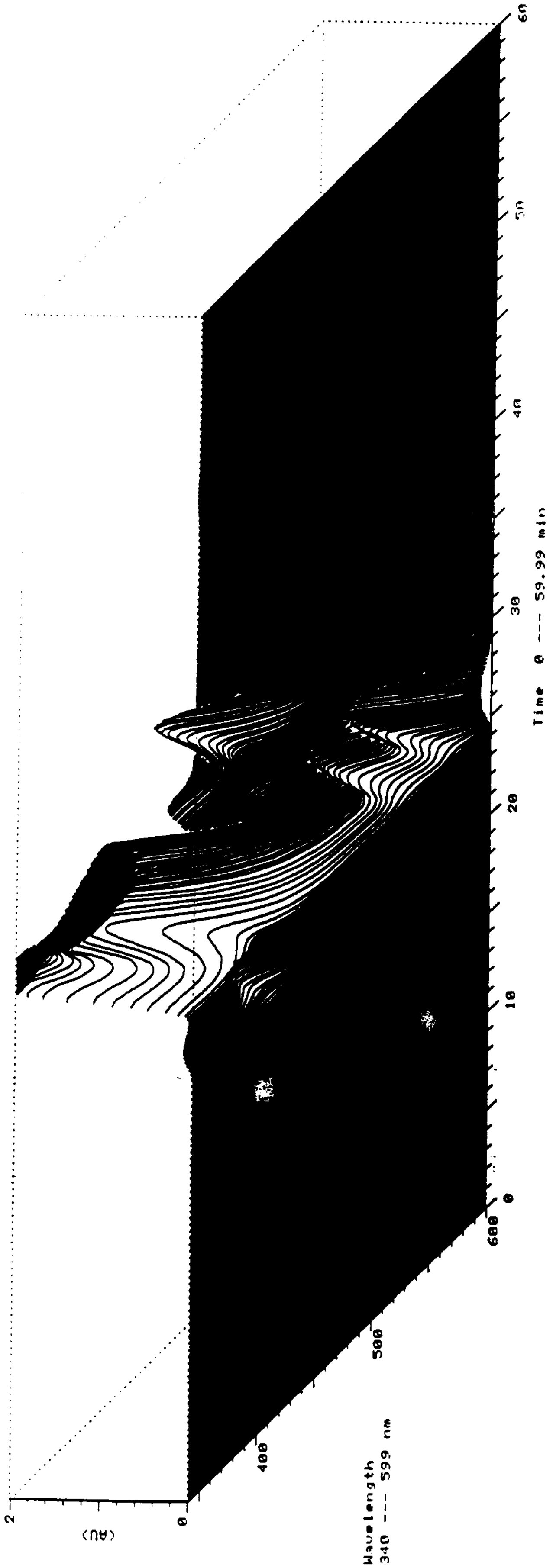
Time 0 --- 59.99 min
0
10
20
30
40
50
60
Wavelength
340 --- 599 nm
400
500
600
(AU)
0
2

FIGURE 2

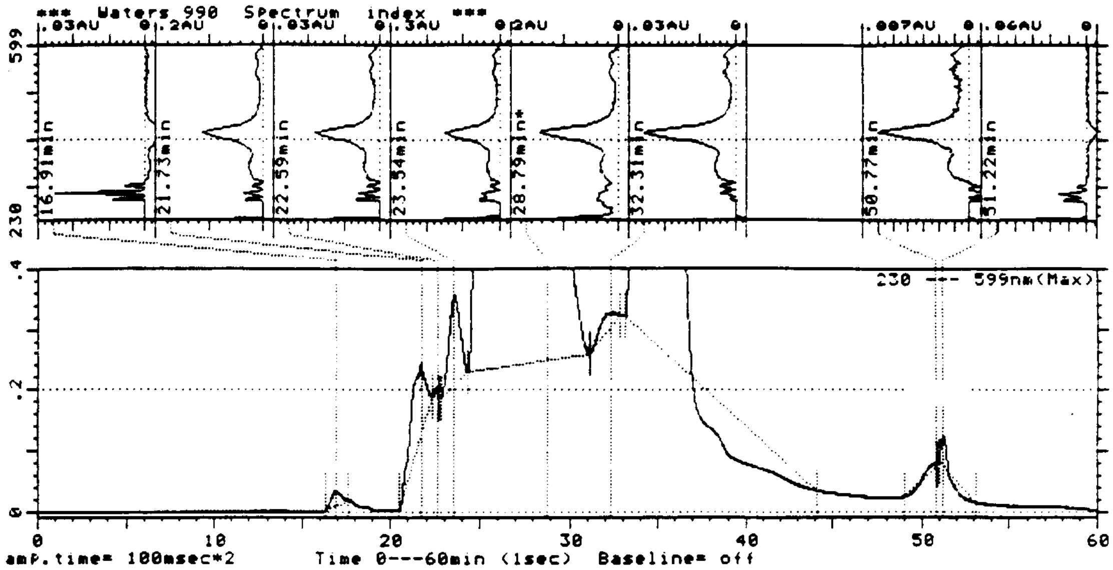
*** Waters 990 Spectrum index ***
230 --- 599nm(Max)
amP.time= 100msec*2
Time 0---60min (1sec)
Baseline= off

Fig. 3 A

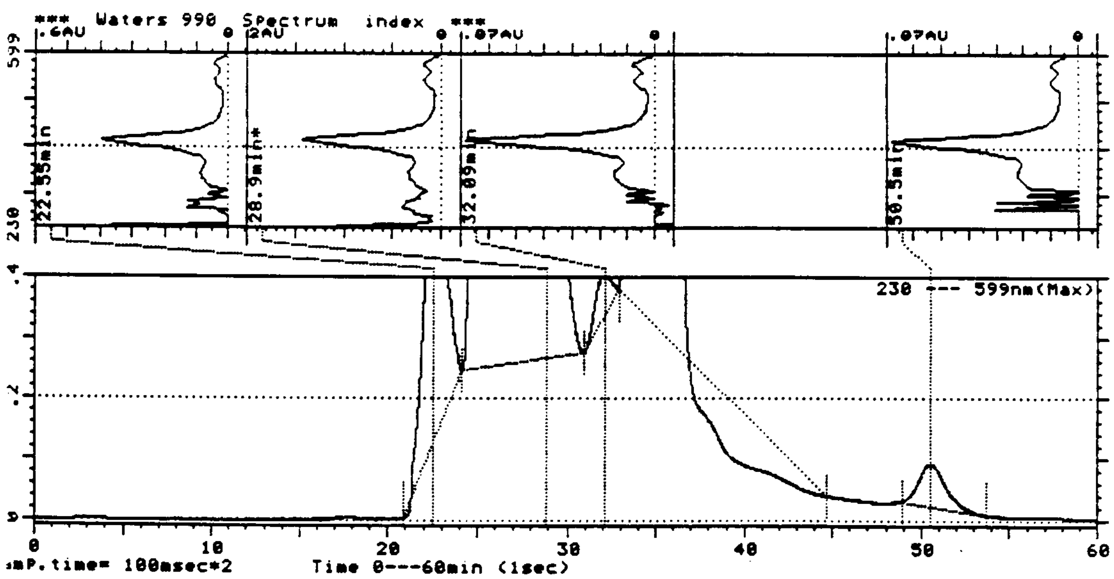
*** Waters 990 Spectrum index ***
230 --- 599nm(Max)
amP.time= 100msec*2
Time 0---60min (1sec)

Fig. 3 B

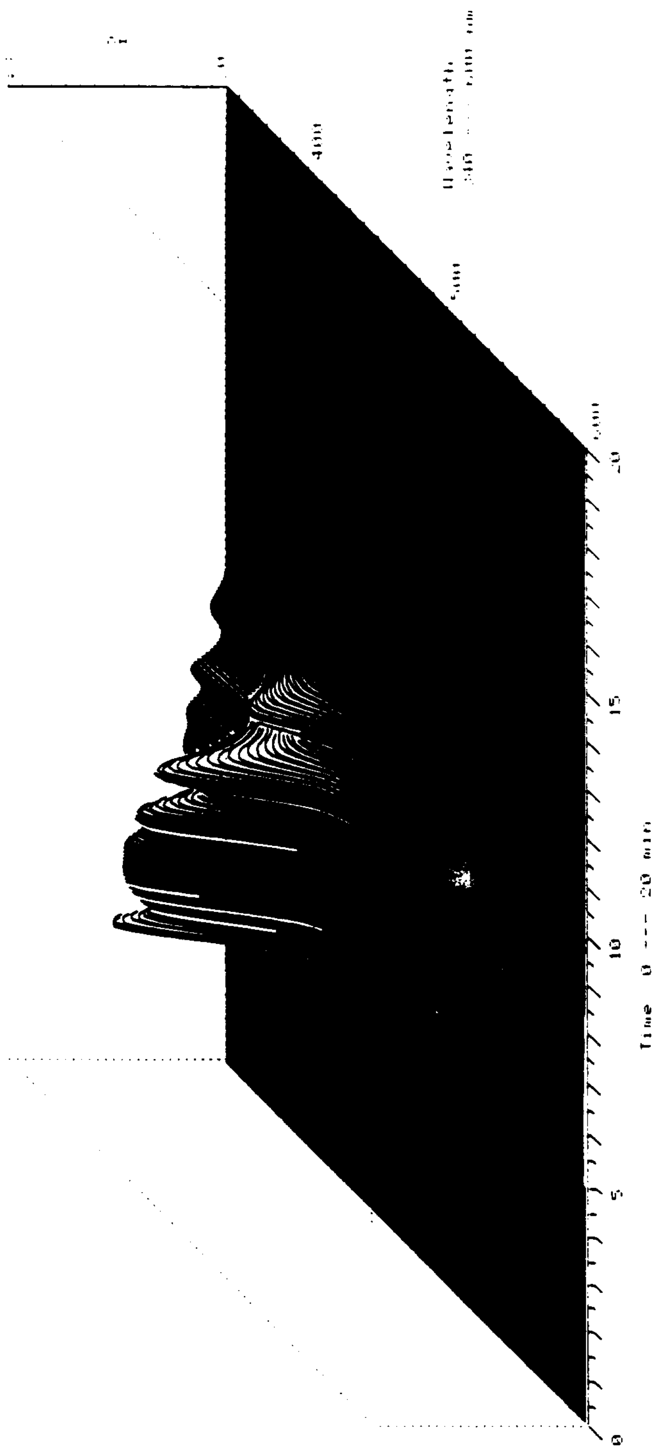

FIGURE 4

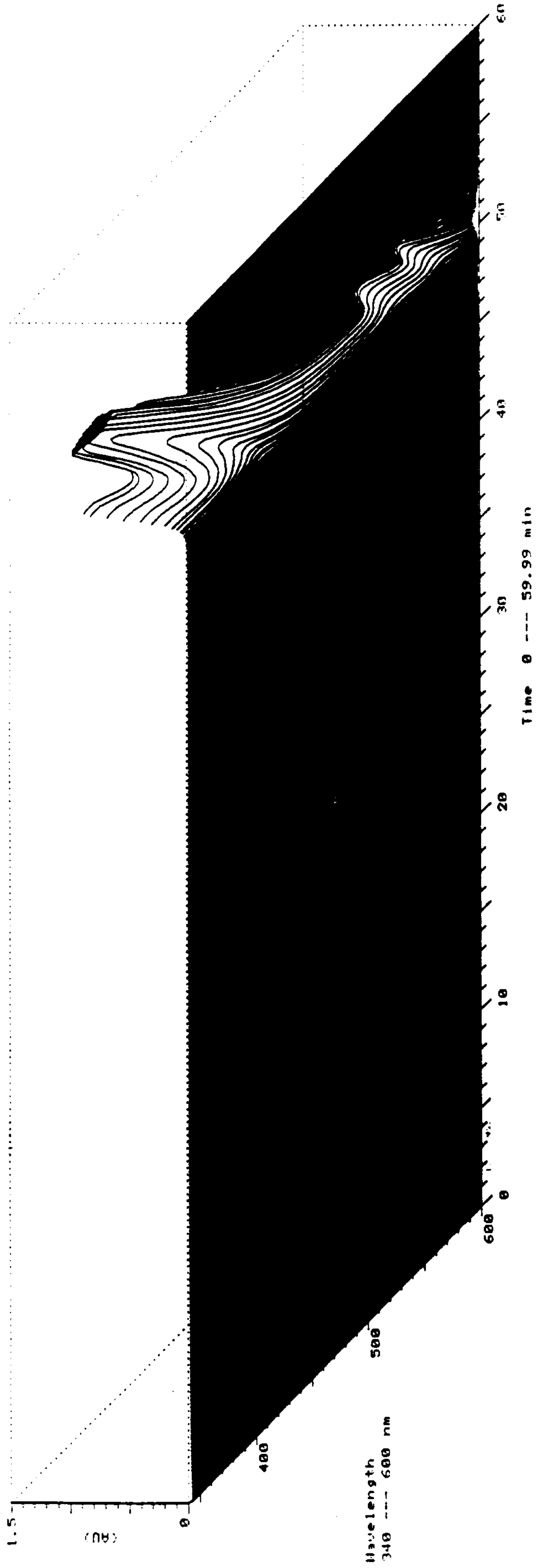
1.5
(AU)
0
400
500
600
Wavelength
340 --- 600 nm
0
10
20
30
40
50
60
Time 0 --- 59.99 min

FIGURE 5

EXAMINATION BY ISOELECTRIC FOCUSING

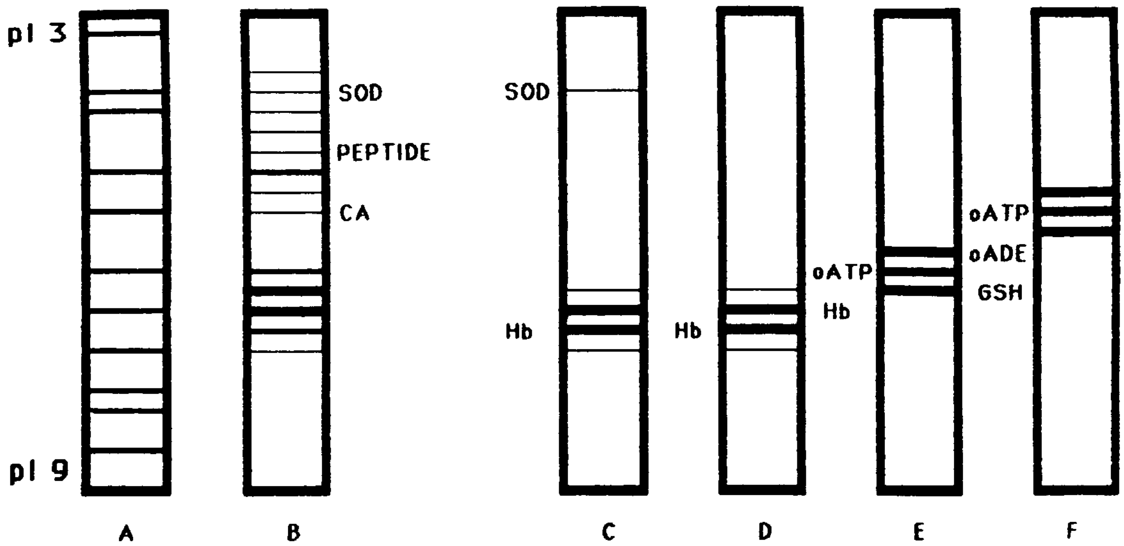


A. Pharmacia Standard, pI 3 - pI 9
B. Hemolysate.
C. Hemoglobin after pasteurization (60 deg.C for 9 hr, and 70 deg.C for 1 hrs)
D. Hemoglobin after centrifugation with chloroform.
E. oATP Hb
F. oATP-oADENOSINE-GSH Hb

---

SOD - Superoxide Dismutase
CA - Carbonic Anhydrase

---

IEF shows both the purification of hemoglobin and the shift of isoelectric point from 6.8 to 6.1.

-FIGURE 6-

**oATP - SEPARATION BY SEPHADEX G-10**

(120 ml SEPHADEX G-10 Column, 0.5 g ATP, Flow 1.5ml/min., Fraction Vol. 3.5 ml)

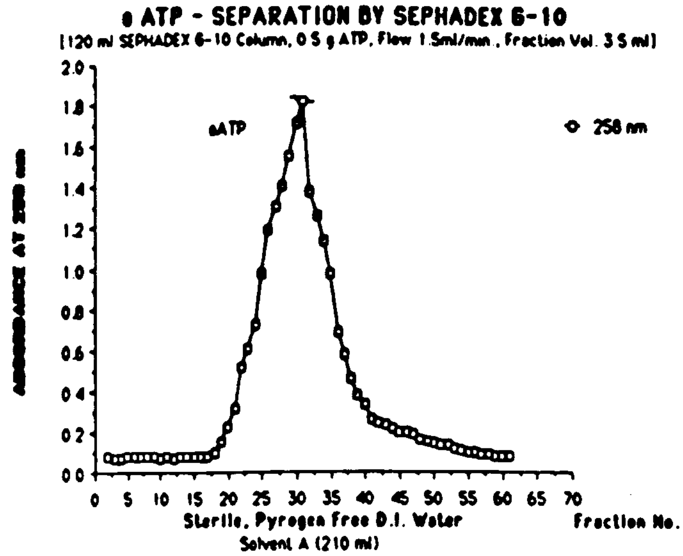


Fig. 7

**PURIFICATION OF ADENOSINE PERIODATE BY ANION EXCHANGE CHROMATOGRAPHY**

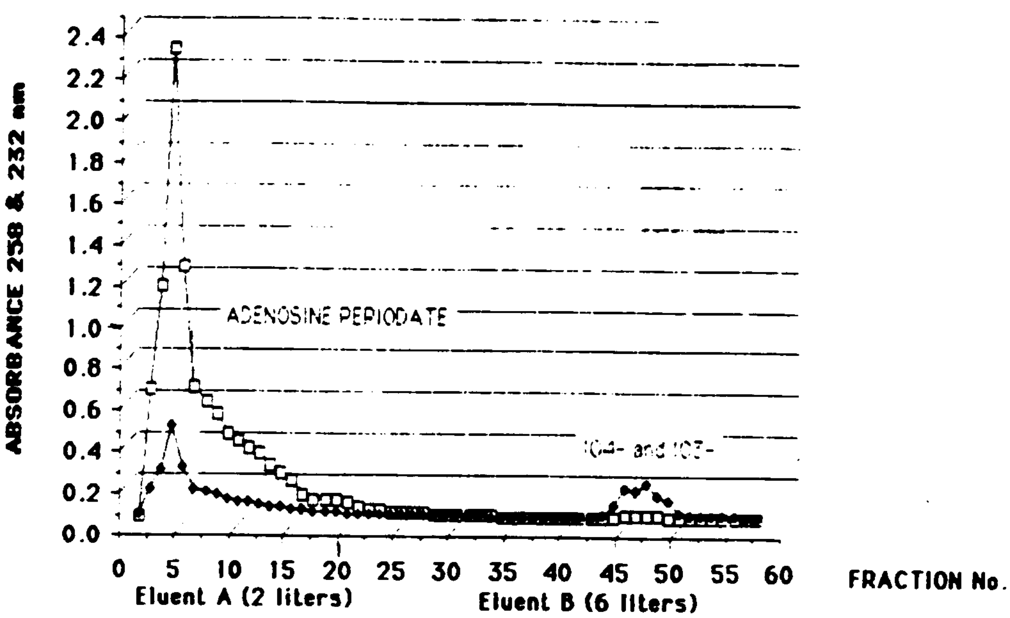


Fig. 8